# EUROPÄISCHE PATENTANMELDUNG

(11) **EP 4 205 710 A1**
(43) Veröffentlichungstag der Anmeldung: **05.07.2023**
(21) Anmeldenummer: 21218350.3
(22) Anmeldetag: 30.12.2021
(51) Int. Cl.: A61F 13/00, A61B 8/08, A61N 7/00, G01N 29/22

(54) **WUNDAUFLAGE FÜR EINE WUNDE**

(71) Anmelder: Paul Hartmann Aktiengesellschaft, 89522 Heidenheim (DE)
(72) Erfinder: Deibler, Martin, 89542 Herbrechtingen (DE); Croizat, Pierre, 89542 Herbrechtingen (DE)
(74) Vertreter: Ter Meer Steinmeister & Partner

(57) **Zusammenfassung**

Die Erfindung betrifft eine Wundauflage zur Behandlung einer Wunde. Die Wundauflage ist auf einen Hautabschnitt eines Menschen oder eines Tieres aufbringbar, wobei der Hautabschnitt die Wunde umfasst. Die Wundauflage umfasst eine erste Anordnung von piezoelektrischen Elementen, wobei zumindest einige der piezoelektrischen Elemente eingerichtet sind, Ultraschall für eine Ultraschallbehandlung der Wunde zu erzeugen.

## Beschreibung

### TECHNISCHES GEBIET

Die Erfindung betrifft eine Wundauflage zur Behandlung einer Wunde. Insbesondere betrifft die Erfindung eine Wundauflage zur Behandlung und Überwachung einer Wunde.

### TECHNISCHER HINTERGRUND

Wundauflagen für Wunden sind bekannt. Dabei wird eine Wundauflage auf eine Wunde zur Wundbehandlung aufgebracht, um diese unter anderem vor äußeren Einflüssen wie Verunreinigungen zu schützen und ein wundheilungsförderndes Milieu hinzuzufügen.

Eine Überprüfung des Heilungsprozesses der Wunde wird in der Regel durch eine Inspektion der Wunde von einem Wundspezialisten durchgeführt. Dazu muss die Wundauflage in regelmäßigen Intervallen von der Wunde entfernt werden und von dem Wundspezialisten bewertet werden. Dadurch kann die Wundruhe gestört werden und somit der Wundheilungsprozess negativ beeinflusst werden.

Es kann notwendig sein, dass ein Patient mit der Wunde den Wundspezialisten mehrfach aufsuchen muss, was für den Patienten belastend ist. Auch werden durch die regelmäßigen Bewertungen durch den Wundspezialisten Ressourcen des Wundspezialisten gebunden.

Eine Quantifizierung und eine objektive Bewertung des Wundheilungsprozesses ist auch durch den Wundspezialisten kaum möglich. Auch eine Bewertung der Effektivität des Therapieansatzes ist nur sehr eingeschränkt möglich.

In der Wunde kann sich ein Biofilm bilden. Biofilme können eine große Herausforderung im Bereich von Wunden darstellen und die Wundheilung beeinträchtigen.

Der Biofilm kann mechanisch entfernt werden. Dazu muss die Wundauflage von der Wunde entfernt werden und von einem Wundspezialisten behandelt werden.

Auch kann es zur Förderung des Heilungsprozesses gewünscht sein, therapeutisch wirksame Substanzen in die Wunde abzugeben, beispielsweise antimikrobielle Substanzen und Medikamente.

### ZUSAMMENFASSUNG DER OFFENBARUNG

Der Erfindung liegt die Aufgabe zu Grunde eine Wundauflage bereitzustellen, die eine verbesserte Behandlung einer Wunde ermöglicht. Eine weitere Aufgabe liegt darin, eine Wundauflage bereitzustellen, durch die eine für die Patienten weniger belastende Behandlung einer Wunde ermöglicht wird. Eine weitere Aufgabe der Erfindung liegt darin, eine Wundauflage bereitzustellen, durch die eine präzise Behandlung eines Biofilms in der Wunde ermöglicht wird. Eine weitere Aufgabe liegt darin, eine Wundauflage bereitzustellen, die einen verbesserten Wundheilungsprozess ermöglicht. Eine weitere Aufgabe liegt darin, eine Wundauflage bereitzustellen, durch die für die Patienten weniger belastende Überwachung eines Wundheilungsprozesses ermöglicht wird. Eine weitere Aufgabe liegt darin, eine Wundauflage bereitzustellen, durch die eine objektive Bewertung eines Wundheilungsprozesses ermöglicht wird.

Gelöst wird zumindest eine der Aufgaben durch die Gegenstände der unabhängigen Ansprüche. Vorteilhafte Weiterbildungen sind in den abhängigen Ansprüchen definiert.

Eine Wundauflage zur Behandlung einer Wunde ist angegeben. Die Wundauflage ist auf einen Hautabschnitt eines Menschen oder eines Tieres aufbringbar. Der Hautabschnitt umfasst die Wunde. Die Wundauflage umfasst eine erste Anordnung von piezoelektrischen Elementen. Zumindest einige der piezoelektrischen Elemente, bevorzugt alle der piezoelektrischen Elemente, sind eingerichtet, Ultraschall für eine Ultraschallbehandlung der Wunde zu erzeugen.

Durch die Fähigkeit der Wundauflage eine Ultraschallbehandlung einer Wunde in einem Hautabschnitt durchzuführen, kann eine Behandlung der Wunde verbessert werden. Die Wundauflage muss nicht von der Wunde entfernt werden, um die Behandlung durchzuführen. Auch muss die Wunde nicht von einem Wundspezialisten behandelt werden. Durch die Wundauflage kann ein Biofilm der Wunde zumindest teilweise zerstört werden. Die Wundauflage kann Mikroorganismen und Biofilm-Fragmente aufnehmen. Durch die Ultraschallbehandlung kann eine Aufnahme von Stoffen durch Zellen in der Wunde ermöglicht oder erhöht werden. Dabei kann die Wundauflage den Stoff bereitstellen.

Allgemein kann eine Wundauflage eine Auflage sein, durch die Eindringen von Fremdkörpern in die Wunde reduziert oder vermieden wird. Die Fremdkörper können Staub, Schmutz oder sonstige Verunreinigungen sein. Die Wundauflage kann ausgebildet sein Blut, Wundexsudat, Mikroorganismen und/oder Biofilm-Fragmente aufzunehmen.

Bevorzugt ist die Wundauflage eine selbsthaftende Wundauflage. Eine selbsthaftende Wundauflage kann auf einen Hautabschnitt aufgeklebt werden, um an dem Hautabschnitt lösbar zu haften. Alternativ kann die Wundauflage nicht selbsthaftend sein, wobei dann zusätzliche Fixierungsmittel, wie z.B. Klebestreifen, erforderlich sind.

Die Wundauflage kann insgesamt flexibel oder nachgiebig ausgestattet sein. Dadurch kann die Wundauflage auf einen unebenen Hautabschnitt aufbringbar sein. Auch kann die Wundauflage eine Bewegung des Hautabschnitts ermöglichen, wenn die Wundauflage auf den Hautabschnitt aufgebracht ist. Beispielsweise kann die Wunde an einem Knie vorliegen. Dazu muss die Wundauflage an der unebenen Haut anliegen können. Auch ist es vorteilhaft, wenn das Körperteil (z.B. das Knie) beweglich bleibt, ohne dass die Wundauflage ihre Haftung zu dem Hautabschnitt des Körperteils verliert. Alternativ kann die Wundauflage unflexibel oder unnachgiebig, beispielsweise starr, sein. Eine starre Wundauflage kann einen zusätzlichen Schutz der Wunde vor einem Eindringen eines Fremdkörpers bereitstellen.

Eine Wunde kann ein Defekt von Deckgewebe der menschlichen oder tierischen Haut sein. Der Defekt kann einen Gewebeverlust umfassen oder keinen Gewebeverlust umfassen.

Der Hautabschnitt, auf den die Wundauflage aufbringbar ist, umfasst die Wunde. Es ist bevorzugt, dass die Wundauflage auf einen Teil des Hautabschnitts außerhalb der Wunde (auch als intakte Haut bezeichnet) und auf die Wunde aufbringbar ist. Die Wundauflage kann eine größere Fläche als die Wunde aufweisen. Die Wunde kann vollständig von der Wundauflage bedeckbar sein.

Die Wundauflage umfasst eine Anordnung von piezoelektrischen Elementen. Ein piezoelektrisches Element kann sich bei einem Anlegen einer elektrischen Spannung verformen. Ebenso kann bei einem piezoelektrischen Element eine elektrische Spannung auftreten, wenn das piezoelektrische Element (elastisch) verformt wird.

Ein piezoelektrisches Element kann einen piezoelektrischen Kristall, eine piezoelektrische Keramik, eine piezoelektrische Dünnschicht und/oder einen piezoelektrischen Kunststoff umfassen oder daraus bestehen. Es ist bevorzugt, dass ein piezoelektrisches Element Polyvinylidenfluorid umfasst oder daraus besteht. Ein piezoelektrisches Element kann ein piezoelektrisches 1-3 Komposit sein. Ein piezoelektrisches Element kann in einer Richtung kontinuierlich ausgebildet sein.

Die Anordnung von piezoelektrischen Elementen kann zumindest ein piezoelektrisches Element umfassen. Bevorzugt umfasst die Anordnung von piezoelektrischen Elementen zumindest zwei, bevorzugter zumindest vier, bevorzugter zumindest sechs, bevorzugter zumindest neun, bevorzugter zumindest 16, bevorzugter zumindest 25, bevorzugter zumindest 36, bevorzugter zumindest 49, piezoelektrische Elemente.

Die Anordnung der piezoelektrischen Elemente kann regelmäßig oder unregelmäßig sein. Beispielsweise können die piezoelektrischen Elemente in Reihen und Spalten angeordnet sein. Piezoelektrische Element in einer Reihe können fluchtend angeordnet sein. Piezoelektrische Element in einer Spalte können fluchtend angeordnet sein. Die Anzahl von Reihen und Spalten kann gleich oder unterschiedlich sein. Die Anzahl von piezoelektrischen Elementen in einer Reihe kann gleich oder unterschiedlich zu der Anzahl von piezoelektrischen Elementen in einer weiteren Reihe der Anordnung sein. Die Anzahl von piezoelektrischen Elementen in einer Spalte kann gleich oder unterschiedlich zu der Anzahl von piezoelektrischen Elementen in einer weiteren Spalte der Anordnung sein.

Zumindest einige der piezoelektrischen Elemente, bevorzugt alle der piezoelektrischen Elemente, sind eingerichtet, Ultraschall zu erzeugen. Ultraschall ist Schall mit einer Frequenz von 20,0 kHz bis 10,0 GHz. Bevorzugt sind die piezoelektrischen Elemente eingerichtet, beispielsweise für eine Ultraschallmessung, Ultraschall in einem Bereich von 0,1 MHz bis 100,0 MHz zu erzeugen, bevorzugter in einem Bereich von 0,5 MHz bis 75,0 MHz, bevorzugter in einem Bereich von 1,0 MHz bis 50,0 MHz, bevorzugter in einem Bereich von 1,0 MHz bis 15 MHz. Die piezoelektrischen Elemente können eingerichtet sein, beispielsweise für eine Ultraschallbehandlung, Schall (Ultraschall), in einem Bereich von 10 kHz bis 1 MHz, bevorzugt von 10 kHz bis 500 kHz, bevorzugter von 10 kHz bis 200 kHz, bevorzugter von 10 kHz bis 100 kHz, bevorzugter von 20 kHz bis 60 kHz, zu erzeugen. Die piezoelektrischen Elemente können eingerichtet sein, beispielsweise für eine Ultraschallbehandlung, Ultraschall in einem Bereich von 20 kHz bis 1 MHz, bevorzugt von 20 kHz bis 500 kHz, bevorzugter von 20 kHz bis 200 kHz, bevorzugter von 20 kHz bis 100 kHz, bevorzugter von 20 kHz bis 60 kHz, zu erzeugen.

Die Ultraschallbehandlung kann ein Erzeugen von Kavitation, insbesondere Mikrokavitation, sein. Die Kavitation kann in der Wunde erzeugt werden. Die Ultraschallbehandlung kann ein zumindest teilweises Zerstören oder zumindest teilweises Lösen eines Biofilms in der Wunde sein. Die Ultraschallbehandlung kann eine Aufnahme von Stoffen durch Zellen, insbesondere durch Zellen in der Wunde, ermöglichen oder erhöhen.

Die piezoelektrischen Elemente können eingerichtet sein, Ultraschall mit einer Schallintensität von 0,05 W/cm² bis 10,0 W/cm², bevorzugt von 0,10 W/cm² bis 5,0 W/cm², bevorzugter von 0,10 W/cm² bis 3,0 W/cm², zu erzeugen.

Die piezoelektrischen Elemente können eingerichtet sein, Ultraschall mit einem Schalldruckpegel von 0,05 MPa bis 10,0 MPa, bevorzugt von 0,10 MPa bis 5,0 MPa, bevorzugter von 0,12 MPa bis 2,4 MPa, zu erzeugen.

Die piezoelektrischen Elemente können so steuerbar sein, dass höchstens einige der piezoelektrischen Elemente Ultraschall für die Ultraschallbehandlung der Wunde erzeugen. Insbesondere können die piezoelektrischen Elemente so steuerbar sein, dass höchstens einige der piezoelektrischen Elemente gleichzeitig Ultraschall für die Ultraschallbehandlung der Wunde erzeugen.

Die piezoelektrischen Elemente können so steuerbar sein, dass nur definierte der piezoelektrischen Elemente Ultraschall für die Ultraschallbehandlung der Wunde erzeugen. Insbesondere können die piezoelektrischen Elemente so steuerbar sein, dass nur definierte der piezoelektrischen Elemente gleichzeitig Ultraschall für die Ultraschallbehandlung der Wunde erzeugen.

Die piezoelektrischen Elemente können so steuerbar sein, dass zumindest zwei der piezoelektrischen Elemente Ultraschall mit unterschiedlicher Leistung für die Ultraschallbehandlung der Wunde erzeugen. Insbesondere sind die piezoelektrischen Elemente so steuerbar, dass zumindest zwei der piezoelektrischen Elemente gleichzeitig Ultraschall mit unterschiedlicher Leistung für die Ultraschallbehandlung der Wunde erzeugen.

Die piezoelektrischen Elemente können so steuerbar sein, dass zu einem Zeitpunkt nur höchstens einige der piezoelektrischen Elemente, bevorzugt höchstens 50 % der piezoelektrischen Elemente, bevorzugter höchstens 10 % der piezoelektrischen Elemente, bevorzugter höchstens eines der piezoelektrischen Elemente, Ultraschall für die Ultraschallbehandlung der Wunde erzeugen.

Die piezoelektrischen Elemente können so steuerbar sein, dass die piezoelektrischen Elemente zeitversetzt Ultraschall für die Ultraschallbehandlung der Wunde erzeugen. Beispielsweise können zu einem ersten Zeitpunkt höchstens einige der piezoelektrischen Elemente Ultraschall für die Ultraschallbehandlung der Wunde erzeugen und zu einem zweiten Zeitpunkt höchstens einige der piezoelektrischen Elemente Ultraschall für die Ultraschallbehandlung der Wunde erzeugen. Die Erzeugungen von Ultraschall zu dem ersten und zweiten Zeitpunkt können für eine oder die gleiche Ultraschallbehandlung der Wunde erzeugt werden.

Die Wundauflage kann eine wundheilende Substanz umfassen, der an die Wunde abgebbar ist. Die Substanz kann in die Wunde einbringbar sein und durch die Ultraschallbehandlung kann eine Aufnahme der Substanz in eine Zelle der Wunde erhöht oder ermöglicht werden. Beispielsweise kann die Substanz aus der Wundauflage in die Wunde diffundieren.

Die Substanz kann in einen Träger eingebracht sein. Der Träger kann die Substanzumgeben und/oder stabilisieren. Der Träger kann eine polymere Mizelle, ein kationisches Lipid, ein kationisches Polymer, ein Liposom und/oder ein Lipidvesikel umfassen. Die polymere Mizelle kann Pluronic P123/F127 umfassen. Das kationische Lipid kann 1,2-Dioctadecanoyl-3-Trimethylammonium-propan (DSTAP) oder 1,2-Distearoyl-3-Trimethylammoniumpropan oder ein Salz, insbesondere ein Chlorid davon, umfassen. Das kationische Polymer kann Polyallylamin-Hydrochlorid umfassen. Das Lipidvesikel kann Lipofectamin oder Lipofectamin 2000 umfassen. Andere Träger sind möglich.

Die Wundauflage kann eine Wundauflage zur Überwachung und Behandlung einer Wunde sein. Die Wundauflage kann eine zweite Anordnung von piezoelektrischen Elementen umfassen. Zumindest einige der piezoelektrischen Elemente der zweiten Anordnung können eingerichtet sein, Ultraschall für eine Ultraschallmessung des Hautabschnitts zu erzeugen. Auf Grundlage der Ultraschallmessung des Hautabschnitts kann eine oder mehrere Eigenschaften der Wunde ermittelbar sein.

Zumindest einige der piezoelektrischen Elemente der zweiten Anordnung können eingerichtet sein, Ultraschall für eine erste Ultraschallmessung des Hautabschnitts innerhalb eines ersten Zeitraums zu erzeugen und Ultraschall für eine zweite Ultraschallmessung des (gleichen) Hautabschnitts innerhalb eines zweiten Zeitraums zu erzeugen. Auf Grundlage der ersten Ultraschallmessung und der zweiten Ultraschallmessung kann die eine oder mehreren Eigenschaften der Wunde ermittelbar sein.

Die zumindest einigen der piezoelektrischen Elemente der zweiten Anordnung können eingerichtet sein, Ultraschall für eine Vielzahl von Ultraschallmessungen des Hautabschnitts innerhalb einer Vielzahl von Zeiträumen zu erzeugen. Auf Grundlage der Vielzahl von Ultraschallmessungen kann die eine oder mehreren Eigenschaften der Wunde ermittelbar sein. Die Vielzahl kann zumindest drei, bevorzugt zumindest fünf, bevorzugter zumindest 10, bevorzugter zumindest 20, sein.

Zwischen zwei Zeiträumen kann ein zeitlicher Abstand von zumindest 5 min, bevorzugt zumindest 15 min, bevorzugter zumindest 1 h, bevorzugter zumindest 1 d (ein Tag), liegen. Das kann für alle hierin offenbarten Zeiträume gelten. Durch mehrere Ultraschallmessungen über einen langen Zeitraum kann eine Entwicklung der einen oder mehreren Eigenschaften der Wunde besonders gut überwacht werden.

Die eine oder mehreren Eigenschaften der Wunde können eine Fläche der Wunde, eine Tiefe der Wunde, ein Heilungsfortschritt der Wunde, eine Heilungskomplikation der Wunde, eine Zustandsänderung der Wunde, eine Dichte der Wunde (beispielsweise ein Dichte von Elementen der Wunde), ein Wassergehalt der Wunde, eine Zusammensetzung der Wunde, Gewebeschichten der Wunde und/oder ein mikrobieller Zustand der Wunde sein. Bevorzugt ist die Eigenschaft der Wunde ein mikrobieller Zustand der Wunde oder eine Zustandsänderung der Wunde.

Die piezoelektrischen Elemente der zweiten Anordnung können eingerichtet sein, Ultraschall für die Ultraschallmessung zu erzeugen und zu empfangen. Insbesondere sind die piezoelektrischen Elemente der zweiten Anordnung eingerichtet, innerhalb eines Zeitraums zeitlich versetzt Ultraschall zu erzeugen und Ultraschall zu empfangen. Die piezoelektrischen Elemente können also eingerichtet sein, als Sender und als Empfänger zu agieren. Dazu kann ein piezoelektrisches Element Ultraschall erzeugen (Sender) und anschließend eine Reflexion des erzeugten Ultraschalls empfangen (Empfänger). Der Zeitraum kann zumindest 1 s oder zumindest 5 s umfassen. Der Zeitraum kann höchstens 1 min oder höchstens 0,5 min umfassen. Das kann für alle hierin offenbarten Zeiträume gelten.

Die Wundauflage kann eine dritte Anordnung von piezoelektrischen Elementen umfassen.

Die piezoelektrischen Elemente der zweiten Anordnung können eingerichtet sein, Ultraschall für die Ultraschallmessung zu erzeugen, und die piezoelektrischen Elemente der dritten Anordnung können eingerichtet sein, Ultraschall für die Ultraschallmessung zu empfangen. Für ein Senden von Ultraschall und für ein Empfangen von Ultraschall können also unterschiedliche piezoelektrische Elemente in der Wundauflage vorgesehen sein.

Bevorzugt sind die piezoelektrischen Elemente der zweiten Anordnung eingerichtet, innerhalb eines ersten Zeitraums Ultraschall zu erzeugen, und die piezoelektrischen Elemente der dritten Anordnung sind eingerichtet, (innerhalb des ersten Zeitraums) Ultraschall zu empfangen. Innerhalb eines zweiten Zeitraums können die piezoelektrischen Elemente der zweiten Anordnung eingerichtet sein, Ultraschall zu empfangen, und die piezoelektrischen Elemente der dritten Anordnung können (innerhalb des zweiten Zeitraums) eingerichtet sein, Ultraschall zu senden. Zunächst können die piezoelektrischen Elemente der zweiten Anordnung als Sender und die piezoelektrischen Elemente der dritten Anordnung als Empfänger agieren, und nachfolgend können die piezoelektrischen Elemente der zweiten Anordnung als Empfänger und die piezoelektrischen Elemente der dritten Anordnung als Sender agieren.

Die piezoelektrischen Elemente der zweiten Anordnung können in einer ersten Ebene angeordnet sein. Die piezoelektrischen Elemente der dritten Anordnung können in einer zweiten Ebene angeordnet sein. Die erste Ebene und die zweite Ebene können übereinander liegend orientiert sein. Die erste Ebene und/oder die zweite Ebene können parallel zu einer von der Wundauflage definierten Ebene orientiert sein.

Bevorzugt sind die piezoelektrischen Elemente der zweiten Anordnung eingerichtet, Ultraschall für die Ultraschallmessung zu erzeugen, und die piezoelektrischen Elemente der dritten Anordnung sind eingerichtet, Ultraschall für die Ultraschallmessung zu empfangen. Besonders bevorzugt sind die piezoelektrischen Elemente der zweiten Anordnung eingerichtet, Ultraschall für die Ultraschallmessung zu erzeugen, und gleichzeitig die piezoelektrischen Elemente der dritten Anordnung eingerichtet, Ultraschall für die Ultraschallmessung zu empfangen. Es kann also vorgesehen sein, dass die piezoelektrischen Elemente der zweiten Anordnung für eine Ultraschallmessung als Sender agieren und (gleichzeitig) die piezoelektrischen Elemente der dritten Anordnung für die Ultraschallmessung als Empfänger agieren.

Die piezoelektrischen Elemente der ersten Anordnung können in einer ersten Ebene angeordnet sein. Die piezoelektrischen Elemente der zweiten und/oder dritten Anordnung können in einer zweiten Ebene angeordnet sein. Die erste Ebene und die zweite Ebene können übereinander liegend orientiert sein. Die erste und/oder zweite Ebene können parallel zu einer von der Wundauflage definierten Ebene orientiert sein.

Die piezoelektrischen Elemente der ersten Anordnung können in einer Ebene mit den piezoelektrischen Elementen der zweiten und/oder dritten Anordnung angeordnet sein. Die Ebene kann parallel zu einer von der Wundauflage definierten Ebene orientiert sein.

Die Leistung der Ultraschallerzeugung für die Ultraschallerzeugung kann an den Zustand der Wunde anpassbar sein. Dadurch kann eine Belastung der Wunde, z.B. Wundschäden, reduziert oder vermieden werden.

Die piezoelektrischen Elemente der ersten Anordnung können eingerichtet sein, Ultraschall mit einer gleichen oder höheren Leistung zu erzeugen als die piezoelektrischen Elemente der zweiten und/oder dritten Anordnung.

Die piezoelektrischen Elemente der zweiten Anordnung und/oder die piezoelektrischen Elemente der dritten Anordnung können eingerichtet sein, Ultraschall für eine Ultraschallbehandlung der Wunde mit einer höheren Leistung zu erzeugen als Ultraschall für eine Ultraschallmessung.

Allgemein kann für eine Ultraschallbehandlung Ultraschall mit einer höheren Leistung erzeugbar sein als für eine Ultraschallmessung. Dazu können für die Ultraschallbehandlung gesonderte piezoelektrische Elemente vorgesehen sein. Alternativ kann ein piezoelektrisches Element oder die piezoelektrischen Elemente eingerichtet sein, Ultraschall mit unterschiedlicher Leistung zu erzeugen. Insbesondere kann ein piezoelektrisches Element oder die piezoelektrischen Elemente eingerichtet sein Ultraschall für eine Ultraschallmessung und Ultraschall für eine Ultraschallbehandlung zu erzeugen. Zusätzlich kann das piezoelektrische Element oder die piezoelektrischen Elemente eingerichtet sein Ultraschall für eine Ultraschallmessung zu empfangen.

Die piezoelektrischen Elemente der ersten Anordnung können eingerichtet sein, Ultraschall für eine Ultraschallbehandlung der Wunde auf Grundlage der einen oder mehreren Eigenschaften der Wunde zu erzeugen.

Alternativ oder zusätzlich können die piezoelektrischen Elemente der zweiten Anordnung und/oder die piezoelektrischen Elemente der dritten Anordnung eingerichtet sein, Ultraschall für eine Ultraschallbehandlung der Wunde auf Grundlage der einen oder mehreren Eigenschaften der Wunde zu erzeugen.

Bevorzugt sind höchstens einige der piezoelektrischen Elemente der ersten, zweiten und/oder dritten Anordnung eingerichtet Ultraschall für eine Ultraschallbehandlung zu erzeugen.

Die Wundauflage kann eine Steuereinheit umfassen. Die Steuereinheit kann eingerichtet sein, die piezoelektrischen Elemente zu steuern. Insbesondere ist die Steuereinheit eingerichtet das Erzeugen und/oder das Empfangen von Ultraschall für die Ultraschallmessung zu steuern. Alternativ oder zusätzlich kann die Steuereinheit eingerichtet sein das Erzeugen von Ultraschall für die Ultraschallbehandlung zu steuern.

Die Wundauflage kann eine Datenübertragungseinrichtung umfassen. Die Datenübertragungseinrichtung kann eingerichtet sein, Daten der Ultraschallmessung an eine Auswerteeinrichtung zu übertragen. Die Datenübertragungseinrichtung kann eingerichtet sein, Daten zu empfangen. Die Datenübertragungseinrichtung kann eine Antenne umfassen. Die Datenübertragungseinrichtung kann eingerichtet sein, Daten drahtlos zu übertragen und/oder zu empfangen. Die Datenübertragungseinrichtung kann eingerichtet sein, Daten über NFC (Near Field Communication), Bluetooth, Bluetooth Low Energy, ZigBee, WLAN (Wireless Local Area Network) und/oder ein Mobilfunknetz zu übertragen.

Die Wundauflage kann eine Energieversorgungseinrichtung umfassen. Die Energieversorgungseinrichtung kann eingerichtet sein, Energie für die Wundauflage bereitzustellen.

Die Energieversorgungseinrichtung kann einen Energiespeicher umfassen. Durch die Energieversorgungseinrichtung kann gespeicherte Energie für die Wundauflage bereitstellbar sein. Die Energieversorgungseinrichtung kann eine Batterie oder einen Akku (Akkumulator) umfassen. Die Batterie kann flexibel oder nachgiebig sein. Bevorzugt ist die Batterie eine Dünnschicht-Batterie. Der Akku kann flexibel oder nachgiebig sein. Bevorzugt ist der Akku ein Dünnschicht-Akku.

Alternativ oder zusätzlich kann die Energieversorgungseinrichtung eingerichtet sein, Energie kontaktbehaftet oder kontaktlos zu empfangen. Die Energieversorgungseinrichtung kann einen Flachstecker umfassen. Über den Flachstecker kann die Energieversorgungseinrichtung mit einer externen Energiequelle, beispielsweise mit einer Steckdose, verbunden werden. Insbesondere ist die Energieversorgungseinrichtung eingerichtet, Energie durch elektromagnetische Strahlung zu empfangen. Die Energieversorgungseinrichtung kann eingerichtet sein, Energie über NFC (Near FieldCommunication), Bluetooth, Bluetooth Low Energy, ZigBee, WLAN (Wireless Local Area Network) und/oder ein Mobilfunknetz zu empfangen.

Die erste, zweite und/oder dritte Anordnung von piezoelektrischen Elementen kann teil- oder vollelastisch, teil- oder vollbiegsam, und/oder teil- oder vollflexibel sein.

Die Wundauflage kann so ausgebildet sein, dass bei einem Aufbringen der Wundauflage auf den Hautabschnitt zumindest einige der piezoelektrischen Elemente der ersten, zweiten und/oder dritten Anordnung von piezoelektrischen Elementen den Hautabschnitt kontaktieren, insbesondere direkt kontaktieren.

Die Fläche der ersten, zweiten und/oder dritten Anordnung von piezoelektrischen Elementen kann größer sein als die Wunde.

Die Wundauflage kann eine Kontaktschicht umfassen. Die Kontaktschicht kann den Hautabschnitt kontaktieren, wenn die Wundauflage auf den Hautabschnitt aufgebracht ist. Die Kontaktschicht kann ein Hydrogel umfassen oder die Kontaktschicht kann eine Hydrogel-Schicht sein.

Die Wundauflage kann eine Schicht umfassen, wobei die Schicht zumindest einige der piezoelektrischen Elemente, bevorzugt alle piezoelektrischen Elemente der Wundauflage, bedeckt oder aufnimmt. Die Schicht kann über der Kontaktschicht angeordnet sein. Die Schicht kann ein Elastomer, insbesondere ein Silikon, umfassen.

Die Wundauflage kann eine Zwischenschicht umfassen. Die Zwischenschicht kann ein absorbierendes Material aufweisen. Alternativ oder zusätzlich kann die Zwischenschicht ein bioaktives Material umfassen. Die Zwischenschicht kann über der Schicht, die zumindest einige der piezoelektrischen Elemente bedeckt oder aufnimmt, angeordnet sein. Die Zwischenschicht kann als Absorptionsschicht, als Adsorptionsschicht oder als Transferschicht ausgeführt sein. Die Zwischenschicht kann ein Fasermaterial, Fleece, ein Vlies, ein Mikrofasermaterial und/oder einen Superabsorber umfassen.

Die Wundauflage kann eine Deckschicht umfassen. Die Deckschicht kann die Wundauflage zumindest teilweise bedecken. Bevorzugt bedeckt die Deckschicht die Wundauflage vollständig. Die Deckschicht kann über der Zwischenschicht angeordnet sein. Die Deckschicht kann elastisch sein.

Die Wundauflage kann eine zumindest teilweise umlaufende Klebeschicht umfassen. Bevorzugt ist die Klebeschicht vollständig umlaufend. Die Deckschicht kann die Klebeschicht bedecken. Durch die Klebeschicht kann die Wundauflage mit dem Hautabschnitt (lösbar) verbunden werden.

Die Wundauflage kann eine Substanz umfassen, der an die Wunde abgebbar ist. Die Kontaktschicht und/oder die Zwischenschicht kann die Substanz umfassen.

Jedes der hierin offenbarten piezoelektrischen Elemente kann eine Abmessung zwischen 0,1 mm und 10,0 mm aufweisen. Jedes der hierin offenbarten piezoelektrischen Elemente kann eine Abmessung zwischen 0,2 mm und 8,0 mm, bevorzugt zwischen 0,4 mm und 6,0 mm, bevorzugter zwischen 0,6 mm und 4,0 mm, bevorzugter zwischen 0,8 mm und 2,0 mm, bevorzugter zwischen 0,9 mm und 1,5 mm, aufweisen. Die Abmessung kann eine Breite und/oder Länge sein. Die Breite und/oder Länge kann sich in einer Ebene erstrecken, die parallel zu einer von der Wundauflage definierten Ebene liegt. Allgemein kann die von der Wundauflage definierte Ebene durch die flächige Erstreckung der Wundauflage definiert werden. Zumindest eines der piezoelektrischen Elemente, bevorzugt zumindest einige der piezoelektrischen Elemente, bevorzugter zumindest 50 % der piezoelektrischen Elemente, bevorzugter alle der piezoelektrischen Elemente der Wundauflage können die Abmessung aufweisen.

Jedes der hierin offenbarten piezoelektrischen Elemente kann eine Fläche zwischen 0,01 mm² und 100,0 mm² aufweisen. Jedes der hierin offenbarten piezoelektrischen Elemente kann eine Fläche zwischen 0,05 mm² und 80,0 mm², bevorzugt zwischen 0,10 mm² und 60,0 mm², bevorzugter zwischen 0,30 mm² und 40,0 mm², bevorzugter zwischen 0,50 mm² und 20,0 mm², bevorzugter zwischen 0,60 mm² und 10,0 mm², bevorzugter zwischen 0,80 mm² und 5,0 mm², aufweisen. Die Fläche kann sich in einer Ebene erstrecken, die parallel zu einer von der Wundauflage definierten Ebene liegt. Die von der Wundauflage definierte Ebene kann durch die flächige Erstreckung der Wundauflage definiert werden. Zumindest eines der piezoelektrischen Elemente, bevorzugt zumindest einige der piezoelektrischen Elemente, bevorzugter zumindest 50 % der piezoelektrischen Elemente, bevorzugter alle der piezoelektrischen Elemente der Wundauflage können die Fläche aufweisen.

Zwischen zwei benachbarten piezoelektrischen Elementen kann ein Abstand zwischen 0,25 mm und 20,0 mm vorliegen. Zwischen zwei benachbarten piezoelektrischen Elementen kann ein Abstand zwischen 0,25 mm und 15,0 mm, bevorzugt zwischen 0,25 mm und 10,0 mm, bevorzugter zwischen 0,25 mm und 5,0 mm, bevorzugter zwischen 0,5 mm und 5,0 mm, bevorzugter zwischen 0,5 mm und 1,0 mm, vorliegen. Der Abstand kann zwischen zwei beliebigen benachbarten piezoelektrischen Elementen vorliegen. Zwischen zwei benachbarten piezoelektrischen Elementen kann kein weiteres piezoelektrisches Element vorliegen. Die benachbarten piezoelektrischen Elemente können piezoelektrische Elemente von unterschiedlichen Anordnungen oder der gleichen Anordnung sein. Der Abstand kann sich in einer Ebene und/oder senkrecht zu einer Ebene erstrecken, die parallel zu einer von der Wundauflage definierten Ebene liegt. Die von der Wundauflage definierte Ebene kann durch die flächige Erstreckung der Wundauflage definiert werden. Zumindest zwischen zwei der piezoelektrischen Elemente, bevorzugt zwischen zumindest einigen der piezoelektrischen Elemente, bevorzugter zwischen zumindest 50 % der piezoelektrischen Elemente, bevorzugter zwischen allen der piezoelektrischen Elemente der Wundauflage kann der Abstand vorliegen.

Zumindest eines der piezoelektrischen Elemente kann die Wunde überlagern und zumindest eines der piezoelektrischen Elemente kann die Wunde nicht überlagern, wenn die Wundauflage auf dem Hautabschnitt aufgebracht ist. Dadurch kann sowohl eine Ultraschallmessung von intakter oder gesunder Haut des Hautabschnitts als auch von der Wunde durchgeführt werden. Die Daten der Ultraschallmessung der intakten oder gesunden Haut kann als Referenz dienen und mit Daten der Ultraschallmessung der Wunde verglichen werden, um die eine oder mehreren Eigenschaften der Wunde zu bestimmen. Die eine oder mehreren Eigenschaften der Wunde können ebenso bestimmt werden, ohne dass Daten der Ultraschallmessung der intakten oder gesunden Haut mit Daten der Ultraschallmessung der Wunde verglichen werden. Intakte oder gesunde Haut kann ein Abschnitt des Hautabschnitts außerhalb der Wunde sein. In dem Abschnitt des Hautabschnitts mit intakter oder gesunder Haut kann keine Wunde vorliegen.

Angegeben ist ein System mit einer Wundauflage und einer Auswerteeinheit. Die Wundauflage kann jede hierin offenbarte Wundauflage sein. Die Auswerteeinheit kann eingerichtet sein, auf Grundlage der Ultraschallmessung des Hautabschnitts die eine oder mehreren Eigenschaften der Wunde zu ermitteln.

Die Auswerteeinheit kann von der Wundauflage beabstandet sein. Beispielsweise kann die Auswerteeinheit ein Server sein. Die Auswerteeinheit kann Daten der Ultraschallmessung erhalten oder empfangen und auf Grundlage der Daten die eine oder mehreren Eigenschaften der Wunde ermitteln. Die Auswerteeinheit kann einen Prozessor umfassen, der eingerichtet ist auf Grundlage der Ultraschallmessung des Hautabschnitts die eine oder mehreren Eigenschaften der Wunde zu ermitteln.

Die Auswerteeinheit kann eine Datenbank umfassen. Die Datenbank kann gespeicherte Ultraschallmessungen umfassen. Zur Ermittlung der einen oder mehreren Eigenschaften der Wunde kann die Auswerteeinheit eine Ultraschallmessung mit den gespeicherten Ultraschallmessungen vergleichen.

Die Auswerteeinheit kann in die Wundauflage integriert sein. Die Wundauflage kann die Auswerteeinheit umfassen.

Allgemein dienen Formulierungen wie erste, zweite und dritte der Unterscheidung von unterschiedlichen Elementen. Sie sind nicht als Aufzählung derart zu verstehen, dass ein erstes und/oder zweites Element vorhanden sein muss, wenn ein drittes Element angesprochen ist. Insbesondere sind Formulierungen wie erste Anordnung von piezoelektrischen Elementen, zweite Anordnung von piezoelektrischen Elementen und dritte Anordnung von piezoelektrischen Elementen nicht so zu verstehen, dass eine erste und/oder zweite Anordnung von piezoelektrischen Elementen vorhanden sein muss, wenn eine dritte Anordnung von piezoelektrischen Elementen angesprochen ist.

Nachfolgend wird anhand von Figuren die Offenbarung bzw. weitere Ausführungsformen und Vorteile der Offenbarung näher erläutert, wobei die Figuren lediglich Ausführungsbeispiele der Erfindung beschreiben. Gleiche Bestandteile in den Figuren werden mit gleichen Bezugszeichen versehen.
Fig. 1 zeigt eine Darstellung einer Wundauflage 100 und einer Auswerteeinheit 500.
Fig. 2 zeigt eine Wundauflage 100 in einer Draufsicht.
Fig. 3 zeigt eine Wundauflage 100 in einer Schnittansicht.
Fig. 4a zeigt eine Wundauflage 100 zu einem ersten Zeitpunkt innerhalb eines ersten Zeitraums t1, wenn die Wundauflage 100 auf einen Hautabschnitt 210 aufgebracht ist.
Fig. 4b zeigt die Wundauflage 100 zu einem zweiten Zeitpunkt innerhalb des ersten Zeitraums t1.
Fig. 4c zeigt die Wundauflage 100 zu einem ersten Zeitpunkt innerhalb eines zweiten Zeitraums t2.
Fig. 4d zeigt die Wundauflage 100 zu einem zweiten Zeitpunkt innerhalb des zweiten Zeitraums t2.
Fig. 5a zeigt eine Wundauflage 100 zu einem ersten Zeitpunkt innerhalb eines ersten Zeitraums t1, wenn die Wundauflage 100 auf einen Hautabschnitt 210 aufgebracht ist.
Fig. 5b zeigt die Wundauflage 100 zu einem zweiten Zeitpunkt innerhalb des ersten Zeitraums t1.
Fig. 5c zeigt die Wundauflage 100 zu einem ersten Zeitpunkt innerhalb eines zweiten Zeitraums t2.
Fig. 5d zeigt die Wundauflage 100 zu einem zweiten Zeitpunkt innerhalb des zweiten Zeitraums t2.
Fig. 6a zeigt eine Wundauflage 100 innerhalb eines ersten Zeitraums t1, wenn die Wundauflage 100 auf einen Hautabschnitt 210 aufgebracht ist.
Fig. 6b zeigt die Wundauflage 100 innerhalb des zweiten Zeitraums t2.
Fig. 7a zeigt eine Wundauflage 100 innerhalb eines ersten Zeitraums t1, wenn die Wundauflage 100 auf einen Hautabschnitt 210 aufgebracht ist.
Fig. 7b zeigt die Wundauflage 100 innerhalb eines zweiten Zeitraums t2.
Fig. 8a zeigt eine Wundauflage 100 zu einem ersten Zeitpunkt innerhalb eines ersten Zeitraums t1, wenn die Wundauflage 100 auf einen Hautabschnitt 210 aufgebracht ist.
Fig. 8b zeigt die Wundauflage 100 zu einem zweiten Zeitpunkt innerhalb des ersten Zeitraums t1.
Fig. 8c zeigt die Wundauflage 100 zu einem Zeitpunkt innerhalb eines zweiten Zeitraums t2.
Fig. 8d zeigt die Wundauflage 100 zu einem Zeitpunkt innerhalb eines dritten Zeitraums t3.
Fig. 9 zeigt eine Wundauflage 100 in einer Draufsicht.

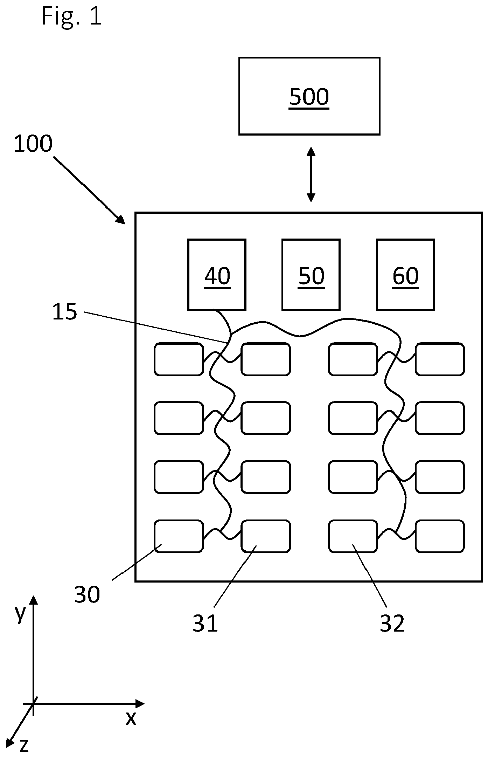

Fig. 1 zeigt eine Wundauflage 100 in einer schematischen Draufsicht. Die flächige Erstreckung der Wundauflage 100 kann eine x-y-Ebene definieren. Abgewinkelt zu der x-y-Ebene, insbesondere senkrecht zu der x-y-Ebene, kann eine z-Richtung orientiert sein.

Allgemein umfasst die Wundauflage 100 zumindest ein piezoelektrisches Element 30, insbesondere mehrere piezoelektrische Elemente 30, 31, 32. Die piezoelektrischen Elemente der Wundauflage 100 können als Anordnung von piezoelektrischen Elementen bezeichnet werden. Die Wundauflage 100 kann mehrere Anordnungen von piezoelektrischen Elementen umfassen. Dabei kann die Aufzählung von Anordnungen von piezoelektrischen Elementen (lediglich) der Unterscheidung dienen. Beispielsweise können piezoelektrische Elemente einer ersten Anordnung, piezoelektrische Elemente einer zweiten Anordnung und/oder piezoelektrische Elemente einer dritten Anordnung auch als piezoelektrische Elemente einer (einzigen) Anordnung verstanden werden, wobei die piezoelektrischen Elemente der (einzigen) Anordnung die piezoelektrischen Elemente der ersten Anordnung, der zweiten Anordnung und/oder der dritten Anordnung umfassen kann.

Die Wundauflage 100 kann piezoelektrische Elemente 30, 31, 32 umfassen. Die piezoelektrischen Elemente 30, 31, 32 können als erste Anordnung von piezoelektrischen Elementen 30, 31, 32 bezeichnet werden. Die piezoelektrischen Elemente 30, 31, 32 der ersten Anordnung können über die Wundauflage 100 verteilt sein. Durch die piezoelektrischen Elemente kann ein Array gebildet sein.

Die piezoelektrischen Elemente 30, 31, 32 sind eingerichtet Ultraschall zu erzeugen. Der Ultraschall kann für eine Ultraschallbehandlung und/oder für eine Ultraschallmessung erzeugbar sein. Dazu kann die Wundauflage 100 auf einen Hautabschnitt 210 eines menschlichen oder tierischen Körpers 200 aufbringbar sein. Der Hautabschnitt 210 umfasst zumindest teilweise, insbesondere vollständig, eine Wunde 300. Für eine Ultraschallbehandlung kann Ultraschall mit einer höheren Leistung erzeugbar sein als für eine Ultraschallmessung. Die piezoelektrischen Elemente 30, 31, 32 können alternativ oder zusätzlich eingerichtet sein, Ultraschall zu empfangen. Der empfangene Ultraschall kann eine Reflexion des von den piezoelektrischen Elementen 30, 31, 32 erzeugten Ultraschalls sein. Die Reflexion kann in dem Körper 200 stattfinden.

Durch die Ultraschallbehandlung kann eine Behandlung der Wunde 300 durchführbar sein. Durch die Ultraschallmessung kann eine oder mehrere Eigenschaften der Wunde 300 ermittelbar sein.

Die Wundauflage 100 kann eine Steuereinheit 40 umfassen. Die Steuereinheit 40 kann eingerichtet sein, die piezoelektrischen Elemente 30, 31, 32 zu steuern. Beispielsweise ist die Steuereinheit 40 eingerichtet, das Erzeugen und/oder Empfangen von Ultraschall durch die piezoelektrischen Elemente 30, 31, 32 zu steuern.

Die Steuereinheit 40 kann eingerichtet sein, einzelne, mehrere oder alle der piezoelektrischen Elemente 30, 31, 32 zu steuern. Insbesondere kann die Steuereinheit 40 eingerichtet sein, einzelne, mehrere oder alle der piezoelektrischen Elemente 30, 31, 32 so zu steuern, dass diese gleichzeitig Ultraschall erzeugen oder empfangen. Beispielsweise kann die Steuereinheit 40 eingerichtet sein, die piezoelektrischen Elemente 30, 31, 32 so zu steuern, dass zu einem ersten Zeitpunkt einige der piezoelektrischen Elemente 30, 31, 32 Ultraschall erzeugen und einige der piezoelektrischen Elemente 30, 31, 32 Ultraschall empfangen. Die Steuereinheit 40 kann eingerichtet sein, die piezoelektrischen Elemente 30, 31, 32 so zu steuern, dass die piezoelektrischen Elemente 30, 31, 32, die zu dem ersten Zeitpunkt Ultraschall erzeugt haben, zu einem zweiten Zeitpunkt Ultraschall empfangen und die piezoelektrischen Elemente 30, 31, 32, die zu dem ersten Zeitpunkt Ultraschall empfangen haben, zu dem zweiten Zeitpunkt Ultraschall erzeugen.

Allgemein können die piezoelektrischen Elemente 30, 31, 32 eingerichtet sein, Ultraschall zu erzeugen und, insbesondere zeitlich beabstandet, Ultraschall zu empfangen. Alternativ oder zusätzlich können die piezoelektrischen Elemente 30, 31, 32 eingerichtet sein, Ultraschall mit einer ersten Leistung zu erzeugen und, insbesondere zeitlich beabstandet, Ultraschall mit einer zweiten Leistung zu erzeugen. Die Ultraschallerzeugung mit der ersten Leistung kann für eine Ultraschallmessung vorgesehen sein. Die Ultraschallerzeugung mit der zweiten Leistung kann für eine Ultraschallbehandlung vorgesehen sein. Die erste Leistung kann geringer sein als die zweite Leistung. Durch die Ultraschallerzeugung mit der zweiten Leistung kann Kavitation in dem Körper 200, insbesondere in der Wunde 300, erzeugbar sein. Durch die Ultraschallerzeugung mit der ersten Leistung kann keine Kavitation in dem Körper 200, insbesondere in der Wunde 300, erzeugbar sein. Die Ultraschallerzeugung und/oder Ultraschallbehandlung kann von der Steuereinheit 40 gesteuert werden. Dazu kann die Steuereinheit 40 die piezoelektrischen Elemente 30, 31, 32 steuern.

Die Steuereinheit 40 kann ein Signal zur Steuerung der piezoelektrischen Elemente 30, 31, 32 erhalten oder empfangen. Das Signal kann von außerhalb der Wundauflage 100 erzeugt werden.

Die Wundauflage 100 kann eine Verbindungsstruktur 15 umfassen. Die Verbindungsstruktur 15 kann mehrere einzelne oder eigenständige Verbindungsstrukturen umfassen. Durch die Verbindungsstruktur 15 können die piezoelektrischen Elemente 30, 31, 32 mit der Steuereinheit 40 verbunden sein. Jedes der piezoelektrischen Elemente 30, 31, 32 kann einzeln oder eigenständig mit der Steuereinheit 40 durch die Verbindungsstruktur 15 verbunden sein.

Bevorzugt ist die Verbindungsstruktur 15 flexibel, biegsam und/oder elastisch ausgestaltet. Dadurch kann die Wundauflage 100 auf einen unebenen Hautabschnitt 210 aufgebracht werden und sogar eine Beweglichkeit des Körperteils mit dem Hautabschnitt 210 ermöglicht werden, ohne dass die Verbindungsstruktur 15 beschädigt wird.

Die Verbindungsstruktur 15 kann eine Dünnschicht-Verbindungsstruktur sein. Die Verbindungsstruktur 15 kann zumindest abschnittsweise meanderförmig ausgebildet sein. Die Verbindungsstruktur 15 kann eine Verdrahtung sein. Die Verbindungsstruktur 15 kann elektrisch leitfähige Fasern, insbesondere elektrisch leitfähige Textilfasern, umfassen. Die Verbindungsstruktur 15 kann zumindest teilweise hergestellt sein durch Transfer-Druck, Mikrolithographie, Ätzen, Laserbearbeitung, Laser-Ablation (Laserverdampfen), Laser-Patterning, Electron-beam evaporation (Elektronenstrahlverdampfung) und/oder Photolithographie.

Die Wundauflage 100 kann eine Energieversorgungseinrichtung 60 umfassen. Die Energieversorgungseinrichtung 60 kann eingerichtet sein, Energie für die Wundauflage 100 bereitzustellen. Die Energieversorgungseinrichtung 60 kann mit der Steuereinheit 40 und/oder mit den piezoelektrischen Elementen 30, 31, 32 verbunden sein, um diese mit Energie zu versorgen. Die Verbindung der Energieversorgungseinrichtung 60 mit der Steuereinheit 40 und/oder mit den piezoelektrischen Elementen 30, 31, 32 kann durch die Verbindungsstruktur 15 bereitgestellt sein. Alternativ kann die Verbindung der Energieversorgungseinrichtung 60 mit der Steuereinheit 40 und/oder mit den piezoelektrischen Elementen 30, 31, 32 durch eine andere Struktur als die Verbindungsstruktur 15 bereitgestellt sein.

Die Energieversorgungseinrichtung 60 kann einen Energiespeicher umfassen, wie eingangs beschrieben. Alternativ oder zusätzlich kann die Energieversorgungseinrichtung eingerichtet sein, Energie kontaktbehaftet oder kontaktlos zu empfangen, wie eingangs beschrieben.

Die Wundauflage 100 kann eine Datenübertragungseinrichtung 50 umfassen. Die Datenübertragungseinrichtung 50 kann eingerichtet sein, Daten und/oder Signale zu empfangen. Datenübertragungseinrichtung 50 kann eingerichtet sein, Daten und/oder Signale zu senden.

Die Datenübertragungseinrichtung 50 kann mit der Steuereinrichtung 40 verbunden sein. Insbesondere ist die Datenübertragungseinrichtung 50 eingerichtet, Daten und/oder Signale (ausgehend) von der Steuereinrichtung 40 zu senden. Die Datenübertragungseinrichtung 50 kann eingerichtet sein, Daten und/oder Signale an die Steuereinrichtung 40 zu übertragen.

Beispielsweise kann die Datenübertragungseinrichtung 50 Daten und/oder Signale zur Steuerung der piezoelektrischen Elemente 30, 31, 32 kontaktlos oder kontaktbehaftet empfangen. Durch die Daten und/oder Signale kann die Steuereinheit 40 die piezoelektrischen Elemente 30, 31, 32 so steuern, dass diese Ultraschall für eine Ultraschallmessung und/oder für eine Ultraschallbehandlung erzeugen.

Die Daten und/oder Signale können von außerhalb der Wundauflage 100 empfangen werden, beispielsweise von einem Server. Die Daten und/oder Signale können von einem entfernten Gerät (z.B. von einem tragbaren Gerät) empfangen werden, beispielsweise von einem Pager, einem Smartphone, einem Tablet und/oder einem Computer. Die Daten und/oder Signale können durch eine Person, beispielsweise einem Träger der Wundauflage und/oder durch medizinisches Personal veranlasst und/oder generiert werden. Die piezoelektrischen Elemente können eingerichtet sein, auf Grundlage der Daten und/oder Signale Ultraschall für eine Ultraschallmessung und/oder eine Ultraschallbehandlung zu erzeugen.

Die Daten und/oder Signale können an einen Ort außerhalb der Wundauflage 100 gesendet werden, beispielsweise an einen oder den Server. Die Daten und/oder Signale können Informationen über die Ultraschallmessung umfassen. Beispielsweise kann eine Ultraschallmessung umfassen, dass zumindest einige der piezoelektrischen Elemente Ultraschall empfangen. Daten und/oder Signale hierzu können, insbesondere über die Steuereinheit 40, von der Datenübertragungseinrichtung 50 an einen Ort außerhalb der Wundauflage 100 gesendet werden. Der Ort kann ein entferntes Gerät (z.B. ein tragbares Gerät) sein. Insbesondere ist das entfernte Gerät ein Pager, ein Smartphone, ein Tablet und/oder ein Computer.

Die Datenübertragungseinrichtung 50 kann mit der Energieversorgungseinrichtung 60 verbunden sein. Die Energieversorgungseinrichtung 60 kann Energie für den Betrieb der Datenübertragungseinrichtung 50 bereitstellen.

Die Datenübertragungseinrichtung 50 kann eine Eingabe-/Ausgabevorrichtung (I/O Interface) sein.

Eine Auswerteeinheit 500 kann in der Wundauflage 100 und/oder außerhalb der Wundauflage 100 vorgesehen sein. Die Auswerteeinheit 500 kann eingerichtet sein, auf Grundlage der Ultraschallmessung des Hautabschnitts 210 die eine oder mehreren Eigenschaften der Wunde 300 zu ermitteln. Die Auswerteeinheit 500 kann eingerichtet sein, Daten und/oder Signale von der Wundauflage 100, insbesondere über die Datenübertragungseinrichtung 50 der Wundauflage 100, zu empfangen. Alternativ oder zusätzlich kann die Auswerteeinheit 500 eingerichtet sein, Daten und/oder Signale an die Wundauflage 100, insbesondere über die Datenübertragungseinrichtung 50 der Wundauflage 100, zu senden.

Beispielsweise kann die Auswerteeinheit 500 eingerichtet sein, Daten und/oder Signale von einer Ultraschallmessung der Wundauflage 100 zu empfangen. Die Auswerteeinheit kann eingerichtet sein, die Daten und/oder Signale zu verarbeiten, um die eine oder mehreren Eigenschaften der Wunde 300 zu ermitteln. Auf Grundlage der einen oder mehreren Eigenschaften der Wunde 300 kann eine Ultraschallbehandlung der Wunde durchführbar sein. Auf Grundlage der einen oder mehreren Eigenschaften der Wunde 300 kann ermittelt werden, ob die Wundauflage von der Wunde 300 entfernt werden soll oder auf der Wunde 300 verbleiben soll. Die Ultraschallbehandlung und/oder die Ermittlung, ob die Wundauflage von der Wunde 300 entfernt werden soll oder auf der Wunde 300 verbleiben soll, kann von der Auswerteeinheit 500 ermittelbar sein. Alternativ oder zusätzlich kann ein Nutzer, bevorzugt der Träger der Wundauflage 100 oder medizinisches Personal, entscheiden, ob eine Ultraschallbehandlung der Wunde 300 und/oder ob die Wundauflage 100 von der Wunde 300 entfernt werden soll oder auf der Wunde 300 verbleiben soll. Eine Ultraschallbehandlung der Wunde 300 durch die Wundauflage 100 kann von dem Nutzer veranlasst werden, bevorzugt durch eine Eingabe.

Die Auswerteeinheit 500 kann eine Datenbank umfassen. In der Datenbank können Daten zu einer Vielzahl von Ultraschallmessungen hinterlegt sein. Auf Grundlage der Vielzahl von Ultraschallmessungen kann die eine oder mehrere Eigenschaften der Wunde 300 ermittelbar sein.

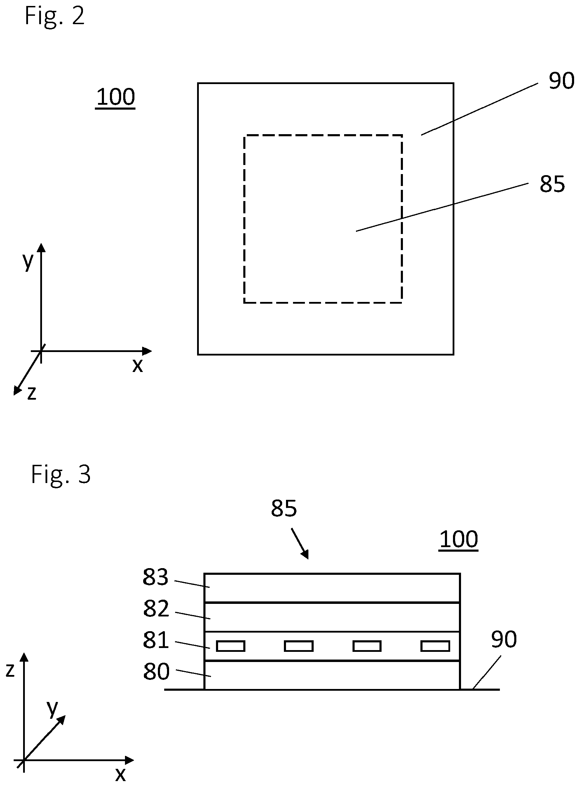

Fig. 2 zeigt schematisch eine Draufsicht der Wundauflage 100. Die Wundauflage 100 kann einen zentralen Abschnitt 85 umfassen. Die Wundauflage 100 kann eine Klebeschicht 90 umfassen. Die Klebeschicht 90 kann ein Kleberand sein. Die Klebeschicht 90 kann die Wundauflage 100 zumindest teilweise, insbesondere vollständig, umgeben oder umlaufen. Bevorzugt umgibt oder umläuft die Klebeschicht 90 den zentralen Abschnitt 85 der Wundauflage 100 zumindest teilweise, insbesondere vollständig.

Durch die Klebeschicht 90 kann die Wundauflage 100 mit dem Hautabschnitt 210 lösbar verbindbar sein. Die Klebeschicht 90 kann außerhalb der Wunde 300 angeordnet werden. Dabei kann die Klebeschicht 90 die Wunde 300 nicht überlagern oder bedecken.

Fig. 3 zeigt die Wundauflage 100 in einer schematischen Schnittansicht.

Die Wundauflage 100 kann eine Deckschicht 83 umfassen. Die Deckschicht 83 kann die Wundauflage 100 zumindest teilweise bedecken. Bevorzugt bedeckt die Deckschicht 83 die Wundauflage vollständig. Die Deckschicht 83 kann elastisch sein. Die Deckschicht 83 kann mit der Klebeschicht 90 verbunden sein oder die Klebeschicht 90 umfassen.

Die Wundauflage 100 kann eine Zwischenschicht 82 umfassen. Die Zwischenschicht 82 kann ein absorbierendes Material aufweisen. Alternativ oder zusätzlich kann die Zwischenschicht ein bioaktives Material umfassen. Die Zwischenschicht 82 kann unter der Deckschicht 83 angeordnet sein. Durch die Zwischenschicht 82 kann Blut, Wundexsudat, Mikroorganismen und/oder Biofilm-Fragmente aufnehmbar sein, insbesondere aus der Wunde 300.

Die Wundauflage 100 kann eine Schicht 81 umfassen, wobei die Schicht 81 zumindest einige der piezoelektrischen Elemente 10, 11, 12, 13, bevorzugt alle piezoelektrischen Elemente 10, 11, 12, 13 der Wundauflage 100, bedeckt oder aufnimmt. Die Schicht 81 kann unter der Zwischenschicht 82 oder über der Zwischenschicht 82 angeordnet sein. Die Schicht 81 kann ein Elastomer, insbesondere ein Silikon, umfassen.

Die Wundauflage 100 kann eine Kontaktschicht 80 umfassen. Die Kontaktschicht 80 kann den Hautabschnitt 210 kontaktieren, wenn die Wundauflage 100 auf den Hautabschnitt 210 aufgebracht ist. Die Kontaktschicht 80 kann ein Hydrogel umfassen oder die Kontaktschicht 80 kann eine Hydrogel-Schicht sein.

Der zentraler Abschnitt 85 der Wundauflage 100 kann die Kontaktschicht 80, die Schicht 81, die zumindest einige der piezoelektrischen Elemente 10, 11, 12, 13 umfasst, die Zwischenschicht 82 und/oder die Deckschicht 83 umfassen. Die Klebeschicht kann außerhalb des zentralen Abschnitts 85 angeordnet sein.

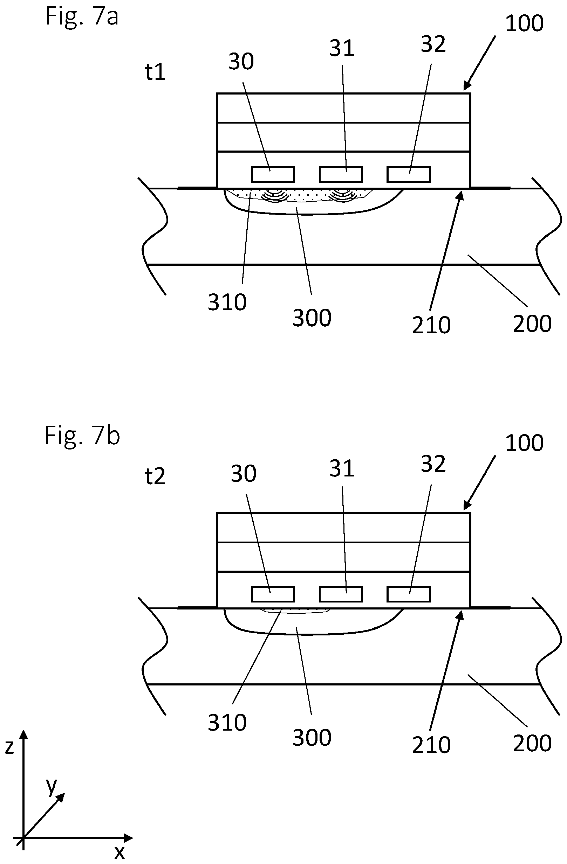

Im Folgenden wird zunächst die Ausführungsform der Fig. 7a und 7b beschrieben.

Fig. 7a zeigt eine Wundauflage 100 innerhalb eines ersten Zeitraums t1, wenn die Wundauflage 100 auf einen Hautabschnitt 210 aufgebracht ist. Dazu ist in Fig. 7a ein Teil eines menschlichen oder tierischen Körpers 200 dargestellt. Der Teil des menschlichen oder tierischen Körpers 200 ist beispielsweise ein Arm. Auf einen Hautabschnitt 210 des Körpers 200 ist eine Wundauflage 100 aufgebracht. Der Hautabschnitt 210 umfasst eine Wunde 300.

Die Wundauflage 100 kann mehrere piezoelektrische Elemente 30, 31, 32, z.B. eine erste Anordnung von piezoelektrischen Elementen 30, 31, 32, umfassen. Bevorzugt ist die Wundauflage 100 so auf den Hautabschnitt 210 aufgebracht, dass zumindest eines der piezoelektrischen Elemente 30, 31, 32 einen intakten oder gesunden Abschnitt des Hautabschnitts 210 überlagert oder bedeckt. In diesem Abschnitt kann keine Wunde 300 vorliegen. Zumindest eines der piezoelektrischen Elemente 30, 31, 32 kann die Wunde 300 überlagern oder bedecken.

Die Wunde 300 kann einen Biofilm 310 umfassen. Der Biofilm 310 kann sich in der Wunde 300 gebildet haben. Das (signifikante) Vorhandensein eines Biofilms 310 kann auf eine Heilungskomplikation der Wunde 300 hindeuten oder eine Heilungskomplikation der Wunde 300 darstellen. Das Vorhandensein eines Biofilms 310 in der Wunde 300 kann durch eine Ultraschallmessung durch die Wundauflage 100 ermittelt werden, wie nachfolgend noch beschrieben wird. Ebenso kann das Vorhandensein eines Biofilms 310 in der Wunde 300 durch eine optische oder olfaktorische Untersuchung, beispielsweise durch einen Wundspezialisten, ermittelt worden sein.

Innerhalb des ersten Zeitraums t1 können zumindest einige der piezoelektrischen Elemente 30, 31, 32 eingerichtet sein, Ultraschall für eine Ultraschallbehandlung zu erzeugen. Durch den Ultraschall für die Ultraschallbehandlung kann Kavitation, insbesondere Mikrokavitation, in der Wunde 300 erzeugt werden. Durch die Kavitation kann der Biofilm 310 zumindest teilweise zerstört oder zumindest teilweise gelöst werden. Die Wundauflage 100 kann Biofilm-Fragmente oder Mikroorganismen aufnehmen. Dadurch kann der Biofilm 310 zumindest teilweise aus der Wunde 300 entfernt werden.

Bevorzugt werden die piezoelektrischen Elemente 30, 31, 32 der Wundauflage 100 so gesteuert, dass höchstens einige der piezoelektrischen Elemente 30, 31, 32, bevorzugt höchstens 50 % der piezoelektrischen Elemente 30, 31, 32, bevorzugter höchstens 10 % der piezoelektrischen Elemente 30, 31, 32, Ultraschall für die Ultraschallbehandlung erzeugen.

Insbesondere können piezoelektrischen Elemente 30, 31, 32, die den Biofilm 310 bedecken oder überlagern, so gesteuert werden, dass diese Ultraschall für die Ultraschallbehandlung erzeugen. Piezoelektrischen Elemente 30, 31, 32, die den Biofilm 310 nicht bedecken oder überlagern, können keinen Ultraschall für die Ultraschallbehandlung erzeugen.

Es ist bevorzugt, dass zu einem ersten Zeitpunkt höchstens einige der piezoelektrischen Elemente 30, 31, 32, bevorzugt höchstens 50 % der piezoelektrischen Elemente 30, 31, 32, bevorzugter höchstens 10 % der piezoelektrischen Elemente 30, 31, 32, die den Biofilm bedecken oder überlagern so gesteuert werden, dass diese Ultraschall für die Ultraschallbehandlung erzeugen. Weitere piezoelektrischen Elemente 30, 31, 32, die den Biofilm 310 bedecken oder überlagern, können so gesteuert werden, dass diese zu einem zweiten Zeitpunkt Ultraschall für die Ultraschallbehandlung erzeugen.

Ein Biofilm 310 in der Wunde 300 muss für eine Ultraschallbehandlung nicht vorhanden sein. Die piezoelektrischen Elemente 30, 31, 32 können unabhängig von einem Vorhandensein eines Biofilms 310 gesteuert werden. Beispielsweise, wenn durch die Ultraschallbehandlung eine Aufnahme einer Substanz ermöglicht oder erhöht werden soll, kann die Ultraschallbehandlung ohne ein Vorhandensein eines Biofilms 310 vorgesehen sein.

Fig. 7b zeigt die Wunde 300 innerhalb eines zweiten Zeitraums t2. In dem zweiten Zeitraum t2 ist ein Teil des Biofilms 310 von der Wundauflage 100 aufgenommen worden. Die Größe des Biofilms 310 ist reduziert. Dadurch kann die Heilung der Wunde 300 verbessert werden.

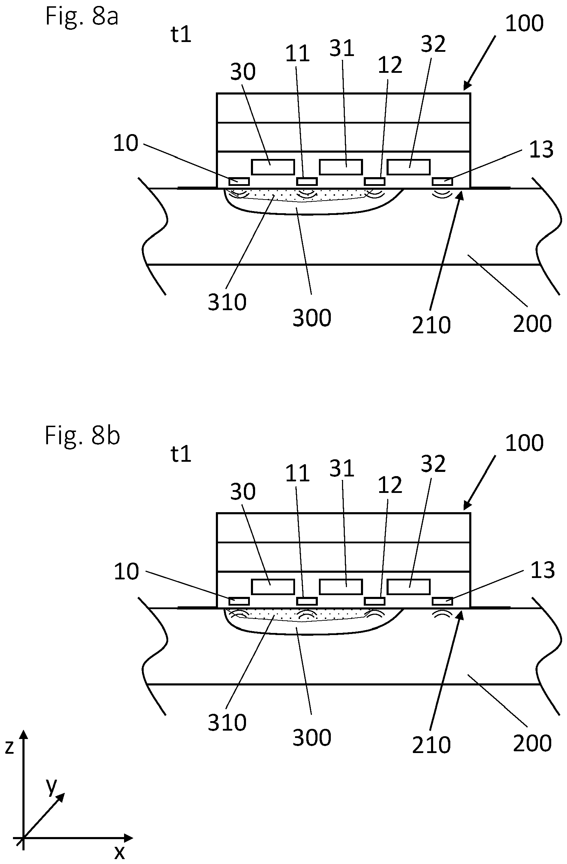

Fig. 8a zeigt die Wundauflage 100 zu einem ersten Zeitpunkt innerhalb eines ersten Zeitraums t1, wenn die Wundauflage 100 auf einen Hautabschnitt 210 aufgebracht ist. Die Wundauflage 100 kann eine Wundauflage 100 zur Überwachung und Behandlung einer Wunde 300 sein.

Die Wundauflage 100 kann piezoelektrische Elemente 30, 31, 32, beispielsweise eine erste Anordnung von piezoelektrischen Elementen 30, 31, 32, umfassen. Die Wundauflage 100 kann piezoelektrische Elemente 10, 11, 12, 13, beispielsweise eine zweite Anordnung von piezoelektrischen Elementen 10, 11, 12, 13, umfassen. Die piezoelektrischen Elemente 30, 31, 32 der ersten Anordnung können ausgebildet sein, eine Ultraschallbehandlung durchzuführen. Die piezoelektrischen Elemente 10, 11, 12, 13 der zweiten Anordnung können ausgebildet sein, eine Ultraschallmessung durchzuführen. Insbesondere können die piezoelektrischen Elemente 30, 31, 32 der ersten Anordnung ausgebildet sein, Ultraschall für eine Ultraschallbehandlung zu erzeugen. Die piezoelektrischen Elemente 10, 11, 12, 13 der zweiten Anordnung können ausgebildet sein, Ultraschall für eine Ultraschallmessung zu erzeugen und zu empfangen.

Die piezoelektrischen Elemente der ersten Anordnung und der zweiten Anordnung werden zur Unterscheidung funktionell unterschieden. Die piezoelektrischen Elemente der ersten Anordnung und der zweiten Anordnung können als piezoelektrische Elemente einer Anordnung oder allgemein als piezoelektrische Elemente der Wundauflage 100 verstanden werden. Allgemein kann jedes der piezoelektrischen Elemente der Wundauflage 100 eingerichtet sein Ultraschall für eine Ultraschallbehandlung zu erzeugen und/oder Ultraschall für eine Ultraschallmessung zu erzeugen und/oder zu empfangen.

Zu dem ersten Zeitpunkt innerhalb des ersten Zeitraums t1 können die piezoelektrischen Elemente 10, 11, 12, 13 der zweiten Anordnung Ultraschall für eine Ultraschallmessung erzeugen. Dazu können die piezoelektrischen Elemente 10, 11, 12, 13 der zweiten Anordnung von der Steuereinheit 40 gesteuert werden. Der von den piezoelektrischen Elemente 10, 11, 12, 13 der zweiten Anordnung erzeugte Ultraschall kann in den Körper 200 eindringen. Dabei kann der Ultraschall in intaktes oder gesundes Gewebe des Körpers 200 eindringen. Dies insbesondere im Bereich des zumindest einen der piezoelektrischen Elemente 10, 11, 12, 13 der zweiten Anordnung, das den intakten oder gesunden Hautabschnitt 210 überlagert oder bedeckt. Zusätzlich kann Ultraschall in die Wunde 300 eindringen. Dies insbesondere im Bereich der piezoelektrischen Elemente 10, 11, 12, 13, die die Wunde 300 überlagern oder bedecken.

In Fig. 8b ist die Wundauflage 100 zu einem zweiten Zeitpunkt innerhalb des ersten Zeitraums t1 dargestellt. Zu dem zweiten Zeitpunkt können die piezoelektrischen Elemente 10, 11, 12, 13 der zweiten Anordnung eingerichtet sein, Ultraschall zu empfangen. Die piezoelektrischen Elemente 10, 11, 12, 13 können von der Steuereinheit 40 gesteuert werden, Ultraschall zu empfangen.

Der von den piezoelektrischen Elementen 10, 11, 12, 13 der zweiten Anordnung empfangene Ultraschall ist insbesondere eine Reflexion des von den piezoelektrischen Elementen 10, 11, 12, 13 der zweiten Anordnung erzeugten Ultraschalls zu dem ersten Zeitpunkt innerhalb des ersten Zeitraums t1. Die Reflexion kann durch unterschiedliche Dichten von verschiedenen Abschnitten des Körpers 200 beeinflusst werden. Insbesondere unterscheidet sich die Reflexion des Ultraschalls in intaktem oder gesundem Gewebe des Körpers und in beschädigtem Gewebe des Körpers (Wunde 300). Ein Umschalten zwischen Erzeugen und Empfangen von Ultraschall kann so durchführbar sein, dass der empfangene Ultraschall reflektierter Ultraschall des erzeugten Ultraschalls ist.

Die Ultraschallmessung kann örtlich aufgelöst sein. Jedes der piezoelektrischen Elemente 10, 11, 12, 13 der zweiten Anordnung kann einem Ort zugeordnet werden oder sein. Die piezoelektrischen Elemente 10, 11, 12, 13 der zweiten Anordnung können einzeln ansteuerbar sein, z.B. von der Steuereinheit 40. Empfangene Ultraschalldaten können einzelnen der piezoelektrischen Elemente 10, 11, 12, 13 der zweiten Anordnung zugeordnet sein oder werden. Die Ultraschallmessung der Wundauflage 100 kann mehrere einzelne Ultraschallmessungen umfassen. Jede der einzelnen Ultraschallmessungen kann durch ein einzelnes piezoelektrisches Element durchführbar sein.

Durch die Ultraschallmessung kann unterscheidbar sein, ob sich an einem Ort des Hautabschnitts 210 unter einem der piezoelektrischen Elemente 10, 11, 12, 13 der zweiten Anordnung eine Wunde 300 oder ein Abschnitt des Hautabschnitts 210 mit intakter oder gesunder Haut befindet. Dazu können die Daten der Ultraschallmessung der piezoelektrischen Elemente 10, 11, 12, 13 der zweiten Anordnung verglichen werden. An einem Abschnitt des Hautabschnitts 210 mit intakter oder gesunder Haut können sich bei der Ultraschallmessung erheblich andere Daten oder Werte ergeben als an einem Abschnitt des Hautabschnitts 210 mit der Wunde. Daten können erheblich anders sein, wenn sich diese signifikant oder auswertbar unterscheiden. Durch einen Vergleich von Daten oder Werten der Ultraschallmessung einzelner piezoelektrischer Elemente 10, 11, 12, 13 der zweiten Anordnung ist keine Kalibrierung der Wundauflage 100 notwendig, wenn zumindest eines der piezoelektrischen Elemente 10, 11, 12, 13 der zweiten Anordnung einen intakten oder gesunden Abschnitt des Hautabschnitts 210 überlagert oder bedeckt, und insbesondere dadurch vermisst.

Auf Grundlage der Ultraschallmessung lässt sich eine Fläche der Wunde, eine Tiefe der Wunde, eines Bereichs der Wunde, ein Heilungsfortschritt der Wunde, eine Heilungskomplikation der Wunde, eine Zustandsänderung der Wunde, eine Dichte der Wunde (beispielsweise eine Dichte von Elementen der Wunde), ein Wassergehalt der Wunde, eine Zusammensetzung der Wunde, Gewebeschichten der Wunde und/oder ein mikrobieller Zustand der Wunde ermitteln. Insbesondere kann durch die Ultraschallmessung das Vorhandensein eines Biofilms 310 festgestellt werden und bevorzugt die Größe des Biofilms 310 ermittelt werden.

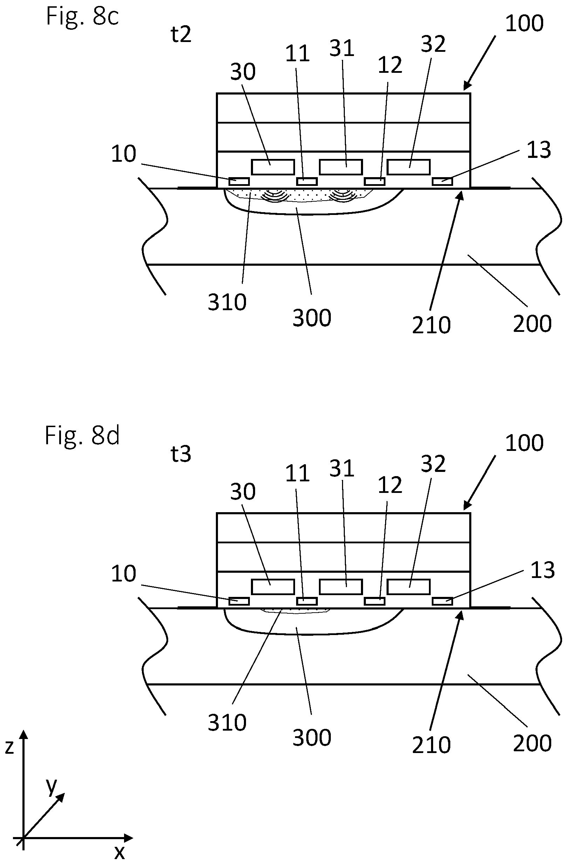

Fig. 8c zeigt die Wundauflage 100 innerhalb eines zweiten Zeitraums t2. Innerhalb des zweiten Zeitraums t2 kann auf Grundlage der Ultraschallmessung innerhalb des ersten Zeitraums t1 eine Ultraschallbehandlung der Wunde 300 vorgesehen sein. Dabei kann jede hierin offenbarte Ultraschallmessung in dem ersten Zeitraum t1 vorgesehen sein. In dem zweiten Zeitraum t2 kann jede hierin offenbarte Ultraschallbehandlung vorgesehen sein.

Die piezoelektrischen Elemente 30, 31, 32 der ersten Anordnung können eingerichtet sein oder so gesteuert werden, Ultraschall für die Ultraschallbehandlung zu erzeugen. Insbesondere werden die piezoelektrischen Elemente 30, 31, 32 der ersten Anordnung so gesteuert, dass nur ausgewählte piezoelektrische Elemente 30, 31, 32 der ersten Anordnung Ultraschall für die Ultraschallbehandlung erzeugen. Eine Auswahl, welche der piezoelektrischen Elemente 30, 31, 32 der ersten Anordnung Ultraschall für die Ultraschallbehandlung erzeugen kann dadurch getroffen sein, ob ein jeweiliges piezoelektrisches Element 30, 31, 32 den Biofilm 310 bedeckt oder überlagert oder nicht. Insbesondere werden nur diejenigen piezoelektrische Elemente 30, 31, 32 der ersten Anordnung so gesteuert, dass diese Ultraschall für die Ultraschallbehandlung erzeugen, wenn diese den Biofilm 310 überlagern oder bedecken.

Durch die Ultraschallmessung kann bestimmt werden, ob und/oder welche der piezoelektrischen Elemente 30, 31, 32 der ersten Anordnung den Biofilm 310 bedecken oder überlagern. Dazu kann die Position der piezoelektrische Elemente 10, 11, 12, 13 der zweiten Anordnung bekannt sein. Durch die Ultraschallmessung kann ermittelt werden, welche der piezoelektrischen Elemente 10, 11, 12, 13 der zweiten Anordnung den Biofilm 310 überlagern oder bedecken. Eine Position von piezoelektrischen Elementen 10, 11, 12, 13 der zweiten Anordnung relativ zu der Position von piezoelektrischen Elementen 30, 31, 32 der ersten Anordnung kann bekannt sein. Dadurch lässt sich ermitteln, ob und/oder welche der piezoelektrischen Elemente 30, 31, 32 der ersten Anordnung den Biofilm 310 bedecken oder überlagern. Alternativ kann jedes der piezoelektrischen Elemente der Wundauflage 100 eingerichtet sein, Ultraschall für eine Ultraschallbehandlung zu erzeugen und/oder Ultraschall für eine Ultraschallmessung zu erzeugen und/oder zu empfangen. In diesem Fall ergibt sich aus der Ultraschallmessungen direkt, welche der piezoelektrischen Elemente den Biofilm 310 bedecken oder überlagern.

Fig. 8d zeigt die Wundauflage 100 innerhalb eines dritten Zeitraums. In dem dritten Zeitraum t3 hat die Größe des Biofilms 310 abgenommen. Die Abnahme der Größe des Biofilms 310 ist durch die Ultraschallbehandlung verursacht. Dadurch kann eine Heilung der Wunde 300 verbessert werden.

Innerhalb des dritten Zeitraums t3 oder nach dem dritten Zeitraum t3 können weitere Ultraschallmessungen und/oder Ultraschallbehandlungen vorgesehen sein. Dadurch lässt sich die Heilung der Wunde 300 weiter verbessern.

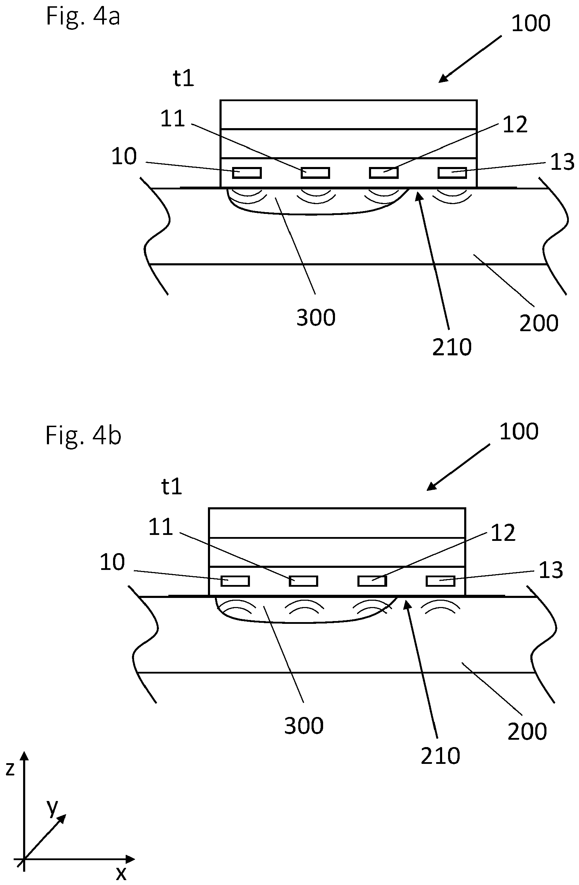

Im Folgenden wird die Ausführungsform der Figs. 4a bis 4d beschrieben.

Fig. 4a zeigt die Wundauflage 100 zu einem ersten Zeitpunkt innerhalb eines ersten Zeitraums t1, wenn die Wundauflage 100 auf einen Hautabschnitt 210 aufgebracht ist. Dazu ist in Fig. 4a ein Teil eines menschlichen oder tierischen Körpers 200 dargestellt. Der Teil des menschlichen oder tierischen Körpers 200 ist beispielsweise ein Arm. Auf einen Hautabschnitt 210 des Körpers 200 ist eine Wundauflage 100 aufgebracht. Der Hautabschnitt 210 umfasst eine Wunde 300.

Die Wundauflage 100 kann mehrere piezoelektrische Elemente 10, 11, 12, 13, z.B. eine zweite Anordnung von piezoelektrischen Elementen 10, 11, 12, 13, umfassen. Bevorzugt ist die Wundauflage 100 so auf den Hautabschnitt 210 aufgebracht, dass zumindest eines der piezoelektrischen Elemente 10, 11, 12, 13 einen intakten oder gesunden Abschnitt des Hautabschnitts 210 überlagert oder bedeckt. In diesem Abschnitt kann keine Wunde 300 vorliegen. Zumindest eines der piezoelektrischen Elemente 10, 11, 12, 13 kann die Wunde 300 überlagern oder bedecken.

Zu dem ersten Zeitpunkt innerhalb des ersten Zeitraums t1 können die piezoelektrischen Elemente 10, 11, 12, 13 Ultraschall für eine Ultraschallmessung erzeugen. Dazu können die piezoelektrischen Elemente 10, 11, 12, 13 von der Steuereinheit 40 gesteuert werden. Der von den piezoelektrischen Elemente 10, 11, 12, 13 erzeugte Ultraschall kann in den Körper 200 eindringen. Dabei kann der Ultraschall in intaktes oder gesundes Gewebe des Körpers 200 eindringen. Dies insbesondere im Bereich des zumindest einen der piezoelektrischen Elemente 10, 11, 12, 13, das den intakten oder gesunden Hautabschnitt 210 überlagert oder bedeckt. Zusätzlich kann Ultraschall in die Wunde 300 eindringen. Dies insbesondere im Bereich der piezoelektrischen Elemente 10, 11, 12, 13, die die Wunde 300 überlagern oder bedecken.

In Fig. 4b ist die Wundauflage 100 zu einem zweiten Zeitpunkt innerhalb des ersten Zeitraums t1 dargestellt. Zu dem zweiten Zeitpunkt können die piezoelektrischen Elemente 10, 11, 12, 13 eingerichtet sein, Ultraschall zu empfangen. Die piezoelektrischen Elemente 10, 11, 12, 13 können von der Steuereinheit 40 gesteuert werden, Ultraschall zu empfangen.

Der von den piezoelektrischen Elementen 10, 11, 12, 13 empfangene Ultraschall ist insbesondere eine Reflexion des von den piezoelektrischen Elementen 10, 11, 12, 13 erzeugten Ultraschalls zu dem ersten Zeitpunkt innerhalb des ersten Zeitraums t1. Die Reflexion kann durch unterschiedliche Dichten von verschiedenen Abschnitten des Körpers 200 beeinflusst werden. Insbesondere unterscheidet sich die Reflexion des Ultraschalls in intaktem oder gesundem Gewebe des Körpers und in beschädigtem Gewebe des Körpers (Wunde 300). Ein Umschalten zwischen Erzeugen und Empfangen von Ultraschall kann so durchführbar sein, dass der empfangene Ultraschall reflektierter Ultraschall des erzeugten Ultraschalls ist.

Daten zu dem von den piezoelektrischen Elementen 10, 11, 12, 13 empfangenen Ultraschall kann an die Auswerteeinheit 500, beispielsweise über die Steuereinheit 40 und/oder die Datenübertragungseinrichtung 50, gesendet werden. Die Energieversorgungseinrichtung 60 kann Energie für das Erzeugen und/oder das Empfangen des Ultraschalls für die Ultraschallmessung bereitstellen.

Allgemein kann eine Ultraschallmessung ein Erzeugen von Ultraschall und ein Empfangen von Ultraschall durch die Wundauflage 100, insbesondere durch die piezoelektrischen Elemente 10, 11, 12, 13 umfassen.

Die Ultraschallmessung kann örtlich aufgelöst sein. Jedes der piezoelektrischen Elemente 10, 11, 12, 13 kann einem Ort zugeordnet werden oder sein. Die piezoelektrischen Elemente 10, 11, 12, 13 können einzeln ansteuerbar sein, z.B. von der Steuereinheit 40. Empfangene Ultraschalldaten können einzelnen der piezoelektrischen Elemente 10, 11, 12, 13 zugeordnet sein oder werden. Die Ultraschallmessung der Wundauflage 100 kann mehrere einzelne Ultraschallmessungen umfassen. Jede der einzelnen Ultraschallmessungen kann durch ein einzelnes piezoelektrisches Element durchführbar sein.

Durch die Ultraschallmessung kann unterscheidbar sein, ob sich an einem Ort des Hautabschnitts 210 unter einem der piezoelektrischen Elemente 10, 11, 12, 13 eine Wunde 300 oder ein Abschnitt des Hautabschnitts 210 mit intakter oder gesunder Haut befindet. Dazu können die Daten der Ultraschallmessung der piezoelektrischen Elemente 10, 11, 12, 13 verglichen werden. An einem Abschnitt des Hautabschnitts 210 mit intakter oder gesunder Haut können sich bei der Ultraschallmessung erheblich andere Daten oder Werte ergeben als an einem Abschnitt des Hautabschnitts 210 mit der Wunde. Daten können erheblich anders sein, wenn sich diese signifikant oder auswertbar unterscheiden. Durch einen Vergleich von Daten oder Werten der Ultraschallmessung einzelner piezoelektrischer Elemente 10, 11, 12, 13 ist keine Kalibrierung der Wundauflage 100 notwendig, wenn zumindest eines der piezoelektrischen Elemente 10, 11, 12, 13 einen intakten oder gesunden Abschnitt des Hautabschnitts 210 überlagert oder bedeckt, und insbesondere dadurch vermisst.

Es kann ebenso vorgesehen sein, dass keines der piezoelektrischen Elemente 10, 11, 12, 13 einen intakten oder gesunden Abschnitt des Hautabschnitts 210 überlagert oder bedeckt. In diesem Fall kann eine Kalibrierung der Wundauflage 100, insbesondere der piezoelektrischen Elemente 10, 11, 12, 13, vorgesehen sein.

Wenn die Wundauflage 100 mehrere piezoelektrische Elemente 10, 11, 12, 13 umfasst, lässt sich die Erstreckung der Wunde 300 in dem Hautabschnitt 210 ermitteln. Beispielsweise kann die Ultraschallmessung ergeben, dass 50 % der piezoelektrischen Elemente 10, 11, 12, 13 die Wunde bedecken oder überlagern. Die Fläche der piezoelektrischen Elemente 10, 11, 12, 13, der Abstand zwischen benachbarten piezoelektrischen Elementen 10, 11, 12, 13 und/oder eine jeweilige Position der piezoelektrischen Elemente 10, 11, 12, 13 in der Wundauflage 100 kann bekannt sein. Dadurch lässt sich die Fläche der Wunde 300 als beispielhafte Eigenschaft der Wunde 300 bestimmen.

Auf Grundlage der Ultraschallmessung lässt sich ebenso eine Tiefe der Wunde, ein Heilungsfortschritt der Wunde, eine Heilungskomplikation der Wunde, eine Zustandsänderung der Wunde, eine Dichte der Wunde (beispielsweise ein Dichte von Elementen der Wunde), ein Wassergehalt der Wunde, eine Zusammensetzung der Wunde, Gewebeschichten der Wunde und/oder ein mikrobieller Zustand der Wunde ermitteln. Dies insbesondere auf Grundlage einer ersten Ultraschallmessung innerhalb eines ersten Zeitraums und einer zweiten Ultraschallmessung innerhalb eines zweiten Zeitraums.

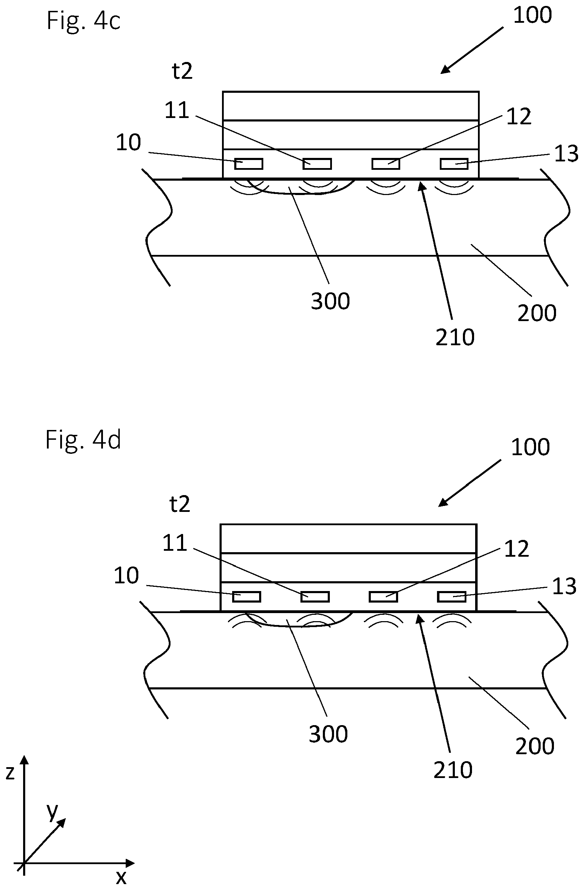

Die mit Blick auf Figs. 4a und 4b beschriebene Ultraschallmessung kann eine erste Ultraschallmessung sein. Die Figs. 4c und 4d zeigen eine Ultraschallmessung innerhalb eines zweiten Zeitraums t2. Die Ultraschallmessung innerhalb des zweiten Zeitraums t2 kann als zweite oder weitere Ultraschallmessung bezeichnet werden. Die Wundauflage 100 kann während des ersten Zeitraums t1 und während des zweiten Zeitraums t2 unverändert auf dem Hautabschnitt 210 aufgebracht sein. Mit anderen Worten, die Position oder die Lage der Wundauflage 100 kann von Beginn des ersten Zeitraums t1 bis Ende des zweiten Zeitraums t2 unverändert auf dem Hautabschnitt 210 sein.

In dem zweiten Zeitraum t2 kann eine zweite Ultraschallmessung durchgeführt werden, die gleich oder analog zu der ersten Ultraschallmessung innerhalb des ersten Zeitraums t1 ist.

Die piezoelektrischen Elemente 10, 11, 12, 13 können zu einem ersten Zeitpunkt innerhalb des zweiten Zeitraums t2 eingerichtet sein, Ultraschall für die Ultraschallmessung zu erzeugen. Zu einem zweiten Zeitpunkt innerhalb des zweiten Zeitraums können die piezoelektrischen Elemente 10, 11, 12, 13 eingerichtet sein, Ultraschall für die Ultraschallmessung zu empfangen.

Die Wunde 300 kann innerhalb des zweiten Zeitraums t2 zumindest teilweise verheilt sein. Beispielsweise kann die Wunde 300 innerhalb des zweiten Zeitraums t2 einer geringeren Fläche aufweisen als innerhalb des ersten Zeitraums t1. Alternativ oder zusätzlich kann die Tiefe der Wunde 300 innerhalb des zweiten Zeitraums t2 geringer sein als innerhalb des ersten Zeitraums t1.

Daten der Ultraschallmessung einzelner piezoelektrischer Elemente 10, 11, 12, 13 der Wundauflage 100 können innerhalb des zweiten Zeitraums t2 unterschiedlich zu Daten der Ultraschallmessung der jeweiligen piezoelektrischen Elemente 10, 11, 12, 13 innerhalb des ersten Zeitraums t1 sein.

Beispielsweise kann innerhalb des zweiten Zeitraums t2 ein größerer Anteil von piezoelektrischen Elementen 10, 11, 12, 13 der Wundauflage 100 einen intakten oder gesunden Abschnitt des Hautabschnitt 210 überlagern oder bedecken als innerhalb des ersten Zeitraums t1. Dies kann durch die erste Ultraschallmessung innerhalb des ersten Zeitraums t1 und durch die zweite Ultraschallmessung innerhalb des zweiten Zeitraums t2 bestimmbar sein. Dadurch kann bestimmt werden, dass die Wunde einen Heilungsfortschritt zeigt.

Ebenso kann innerhalb des zweiten Zeitraums t2 ein geringerer Anteil von piezoelektrischen Elementen 10, 11, 12, 13 der Wundauflage 100 einen intakten oder gesunden Abschnitt des Hautabschnitt 210 überlagern oder bedecken als innerhalb des ersten Zeitraums t1. Dadurch kann bestimmt werden, dass die Wunde eine Heilungskomplikation zeigt.

Auch kann innerhalb des zweiten Zeitraums t2 an einer Position oder Lage eines oder mehrerer der piezoelektrischen Elemente 10, 11, 12, 13 eine andere Zusammensetzung der Wunde 300 vorliegen als innerhalb des ersten Zeitraums t1. Dadurch kann bestimmt werden, dass die Wunde eine Heilungskomplikation zeigt. Beispielsweise kann ein Biofilm in der Wunde gebildet worden sein, wodurch sich die Zusammensetzung der Wunde 300 verändert hat. Alternativ kann dadurch bestimmt werden, dass die Wunde einen Heilungsfortschritt zeigt. Beispielsweise kann ein Biofilm in der Wunde zurückgebildet worden sein, wodurch sich die Zusammensetzung der Wunde 300 verändert hat.

Zur Überwachung der Wunde 300 können eine Vielzahl von Ultraschallmessungen durch die Wundauflage 100 durchführbar sein. Die Überwachung der Wunde 300 kann über einen langen Zeitraum, beispielsweise zumindest 1 Tag, zumindest 3 Tage oder zumindest 1 Woche, durchführbar sein.

Allgemein kann zwischen zwei Zeiträumen ein zeitlicher Abstand von zumindest 5 min, bevorzugt zumindest 15 min, bevorzugter zumindest 1 h, bevorzugter zumindest 1 d (ein Tag), liegen. Es können eine Vielzahl von Zeiträumen betrachtet werden, beispielsweise zumindest fünf, zumindest 10 oder zumindest 20 Zeiträume.

Alle piezoelektrischen Elemente 10, 11, 12, 13 der Wundauflage 100 können eingerichtet sein, zu einem ersten Zeitpunkt Ultraschall für eine Ultraschallmessung zu erzeugen. Alle piezoelektrischen Elemente 10, 11, 12, 13 der Wundauflage 100 können eingerichtet sein, zu einem zweiten Zeitpunkt Ultraschall für eine Ultraschallmessung zu empfangen. Mit anderen Worten, alle piezoelektrischen Elemente 10, 11, 12, 13 der Wundauflage 100 können eingerichtet sein gleichzeitig entweder Ultraschall für eine Ultraschallmessung zu erzeugen oder zu empfangen. Zwischen dem Erzeugen und dem Empfangen von Ultraschall kann ein Umschalten der Funktion oder Arbeitsweise der piezoelektrischen Elemente 10, 11, 12, 13 durchführbar sein. Die piezoelektrischen Elemente 10, 11, 12, 13 können von der Steuereinheit 40 steuerbar sein.

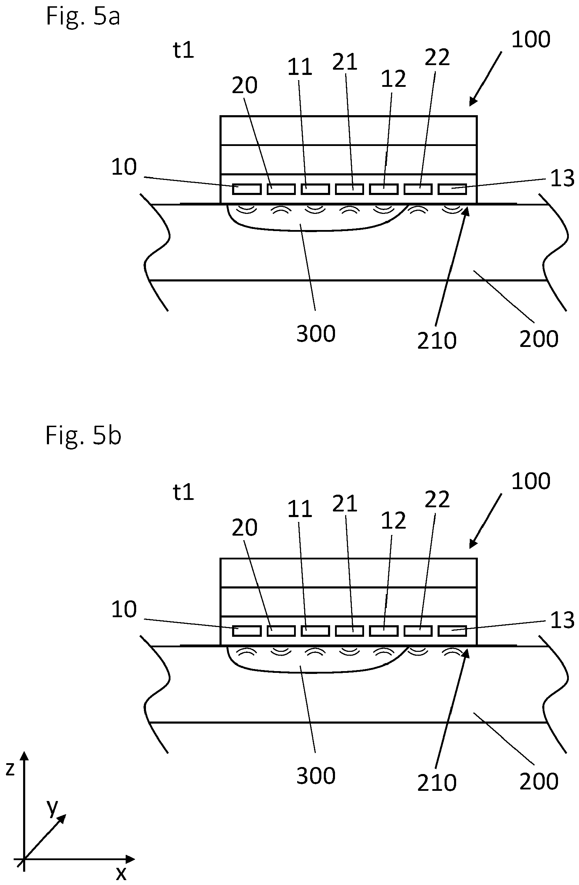

Fig. 5a zeigt eine Wundauflage 100 zu einem ersten Zeitpunkt innerhalb eines ersten Zeitraums t1, wenn die Wundauflage 100 auf einen Hautabschnitt 210 aufgebracht ist. Die Wundauflage 100 ist analog zu den bereits beschriebenen Wundauflagen 100. Im Folgenden werden Unterschiede der Wundauflage zu bereits beschriebenen Wundauflagen 100 beschrieben.

Die Wundauflage 100 kann mehrere piezoelektrische Elemente 10, 11, 12, 13 umfassen, die als zweite Anordnung von piezoelektrischen Elementen 10, 11, 12, 13 bezeichnet werden können. Weiterhin kann die Wundauflage 100 mehrere piezoelektrische Elemente 20, 21, 22 umfassen, die als dritte Anordnung von piezoelektrischen Elementen 20, 21, 22 bezeichnet werden können. Die Beschreibung einer zweiten Anordnung von piezoelektrischen Elementen und einer dritten Anordnung von piezoelektrischen Elementen dient der Unterscheidung. Alle piezoelektrischen Elemente 10, 11, 12, 13, 20, 21, 22 sind in der Wundauflage 100 vorgesehen. Die piezoelektrischen Elemente 10, 11, 12, 13, 20, 21, 22 könnten auch als eine Anordnung von piezoelektrischen Elementen bezeichnet werden.

Die Wundauflage 100 kann so auf den Hautabschnitt 210 aufbringbar sein, dass zumindest eines der piezoelektrischen Elemente 10, 11, 12, 13 der zweiten Anordnung oder zumindest eines der piezoelektrischen Elemente 20, 21, 22 der dritten Anordnung einen intakten oder gesunden Abschnitt des Hautabschnitts 210 überlagert oder bedeckt. In diesem Abschnitt kann keine Wunde 300 vorliegen. Zumindest eines der piezoelektrischen Elemente 10, 11, 12, 13 der zweiten Anordnung oder zumindest eines der piezoelektrischen Elemente 20, 21, 22 der dritten Anordnung kann die Wunde 300 überlagern oder bedecken.

Zu dem ersten Zeitpunkt innerhalb des ersten Zeitraums t1 können die piezoelektrischen Elemente 10, 11, 12, 13 der zweiten Anordnung eingerichtet sein, Ultraschall für eine Ultraschallmessung zu erzeugen. Ebenfalls zu dem ersten Zeitpunkt innerhalb des ersten Zeitraums t1 können die piezoelektrischen Elemente 20, 21, 22 der dritten Anordnung eingerichtet sein, Ultraschall für eine Ultraschallmessung zu empfangen. Allgemein kann zu einem Zeitpunkt ein Anteil der piezoelektrischen Elemente eingerichtet sein, Ultraschall zu erzeugen, und ein anderer Anteil der piezoelektrischen Elemente kann eingerichtet sein, Ultraschall zu empfangen. Bevorzugt sind die piezoelektrischen Elemente der Wundauflage 100 zu einem Zeitpunkt entweder eingerichtet, Ultraschall zu erzeugen oder zu empfangen.

In Fig. 5b ist die Wundauflage 100 zu einem zweiten Zeitpunkt innerhalb des ersten Zeitraums t1 dargestellt. Zu dem zweiten Zeitpunkt können die piezoelektrischen Elemente 10, 11, 12, 13 der zweiten Anordnung eingerichtet sein, Ultraschall für eine Ultraschallmessung zu empfangen. Ebenfalls zu dem zweiten Zeitpunkt innerhalb des ersten Zeitraums t1 können die piezoelektrischen Elemente 20, 21, 22 der dritten Anordnung eingerichtet sein, Ultraschall für eine Ultraschallmessung zu erzeugen.

Bevorzugt kann zwischen dem ersten Zeitpunkt innerhalb des ersten Zeitraums t1 und dem zweiten Zeitpunkt innerhalb des ersten Zeitraums t1 die Funktion oder Arbeitsweise der piezoelektrischen Elemente 10, 11, 12, 13 der zweiten Anordnung und der piezoelektrischen Elemente 20, 21, 22 der dritten Anordnung von einem Erzeugen von Ultraschall zu einem Empfangen von Ultraschall bzw. von einem Empfangen von Ultraschall zu einem Erzeugen von Ultraschall verändert werden. Die piezoelektrischen Elemente, die zu dem ersten Zeitpunkt eingerichtet waren, Ultraschall zu erzeugen, können zu dem zweiten Zeitpunkt eingerichtet sein, Ultraschall zu empfangen. Die piezoelektrischen Elemente, die zu dem ersten Zeitpunkt eingerichtet waren, Ultraschall zu empfangen, können zu dem zweiten Zeitpunkt eingerichtet sein, Ultraschall zu erzeugen. Die piezoelektrischen Elemente der Wundauflage 100 können von der Steuereinheit 40 gesteuert werden.

Zwischen zwei piezoelektrischen Elementen 10, 11, 12, 13 der zweiten Anordnung kann ein piezoelektrisches Element 20, 21, 22 der dritten Anordnung angeordnet sein. Alternativ oder zusätzlich kann zwischen zwei piezoelektrischen Elementen 20, 21, 22 der dritten Anordnung ein piezoelektrisches Element 10, 11, 12, 13 der zweiten Anordnung angeordnet sein. Piezoelektrische Elemente 10, 11, 12, 13 der zweiten Anordnung können alternierend mit piezoelektrischen Elementen 20, 21, 22 der dritten Anordnung angeordnet sein. Die piezoelektrischen Elemente 10, 11, 12, 13 der zweiten Anordnung und die piezoelektrischen Elemente 20, 21, 22 der dritten Anordnung können in einer Ebene angeordnet sein. Die Ebene kann parallel zu einer von der Wundauflage 100 definierten Ebene orientiert sein.

Die zweite Anordnung von piezoelektrischen Elementen 10, 11, 12, 13 kann die gleiche Anzahl von piezoelektrischen Elementen umfassen wie die dritte Anordnung von piezoelektrischen Elementen 20, 21, 22. Alternativ kann die Anzahl unterschiedlich sein.

Die Ultraschallmessung innerhalb des ersten Zeitraums kann als erste Ultraschallmessung bezeichnet werden.

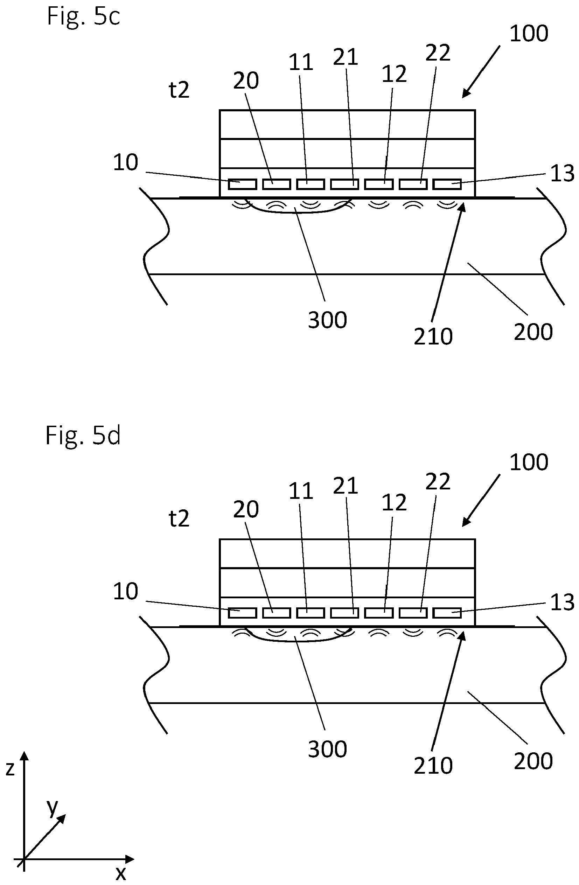

Die Figs. 5c und 5d zeigen eine Ultraschallmessung innerhalb eines zweiten Zeitraums t2. Die Ultraschallmessung innerhalb des zweiten Zeitraums t2 kann als zweite oder weitere Ultraschallmessung bezeichnet werden. Die Position oder die Lage der Wundauflage 100 kann von Beginn des ersten Zeitraums t1 bis Ende des zweiten Zeitraums t2 unverändert auf dem Hautabschnitt 210 sein.

In dem zweiten Zeitraum t2 kann eine zweite Ultraschallmessung durchgeführt werden, die gleich oder analog zu der ersten Ultraschallmessung innerhalb des ersten Zeitraums t1 ist.

In dem zweiten Zeitraum t2, in dem die zweite Ultraschallmessung durchführbar ist, hat sich die Wunde 300 in dem Hautabschnitt 210 analog oder gleich zu der Veränderung der Wunde 300 wie mit Blick auf die Figs. 4a bis 4d beschrieben verändert.

Durch die erste und zweite Ultraschallmessung lässt sich eine Veränderung der Wunde 300 ermitteln. Beispielsweise ist ein Heilungsfortschritt und/oder eine Heilungskomplikation ermittelbar.

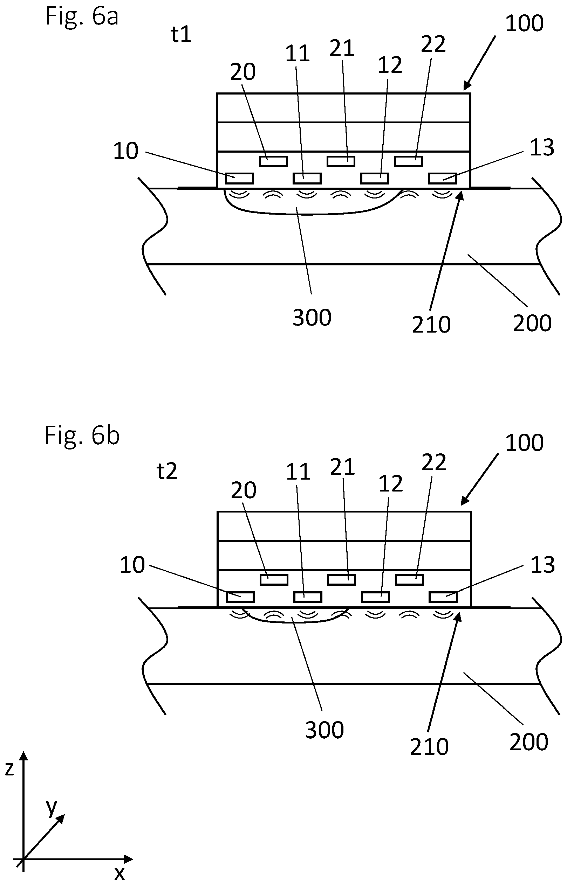

Fig. 6a zeigt eine Wundauflage 100 zu einem ersten Zeitpunkt innerhalb eines ersten Zeitraums t1, wenn die Wundauflage 100 auf einen Hautabschnitt 210 aufgebracht ist. Die Wundauflage 100 ist analog zu den bereits beschriebenen Wundauflagen 100. Im Folgenden werden Unterschiede der Wundauflage zu bereits beschriebenen Wundauflagen 100 beschrieben.

Ähnlich wie die in den Figs. 5a bis 5d gezeigte Wundauflage 100 kann die Wundauflage 100 mehrere piezoelektrische Elemente 10, 11, 12, 13 umfassen, die als zweite Anordnung von piezoelektrischen Elementen 10, 11, 12, 13 bezeichnet werden können. Weiterhin kann die Wundauflage 100 mehrere piezoelektrische Elemente 20, 21, 22 umfassen, die als dritte Anordnung von piezoelektrischen Elementen 20, 21, 22 bezeichnet werden können. Die Beschreibung einer zweiten Anordnung von piezoelektrischen Elementen und einer dritten Anordnung von piezoelektrischen Elementen dient wiederum der Unterscheidung. Alle piezoelektrischen Elemente 10, 11, 12, 13, 20, 21, 22 sind in der Wundauflage 100 vorgesehen. Die piezoelektrischen Elemente 10, 11, 12, 13, 20, 21, 22 könnten auch als eine Anordnung von piezoelektrischen Elementen bezeichnet werden.

Zu dem ersten Zeitpunkt innerhalb des ersten Zeitraums t1 können die piezoelektrischen Elemente 10, 11, 12, 13 der zweiten Anordnung eingerichtet sein, Ultraschall für eine Ultraschallmessung zu erzeugen. Ebenfalls zu dem ersten Zeitpunkt innerhalb des ersten Zeitraums t1 können die piezoelektrischen Elemente 20, 21, 22 der dritten Anordnung eingerichtet sein, Ultraschall für eine Ultraschallmessung zu empfangen. Allgemein kann zu einem Zeitpunkt ein Anteil der piezoelektrischen Elemente eingerichtet sein, Ultraschall zu erzeugen, und ein anderer Anteil der piezoelektrischen Elemente kann eingerichtet sein, Ultraschall zu empfangen. Bevorzugt sind die piezoelektrischen Elemente der Wundauflage 100 zu einem Zeitpunkt entweder eingerichtet, Ultraschall zu erzeugen oder zu empfangen.

Zu dem einem Zeitpunkt innerhalb des ersten Zeitraums t1 können die piezoelektrischen Elemente 10, 11, 12, 13 der zweiten Anordnung eingerichtet sein, Ultraschall für eine Ultraschallmessung zu empfangen. Ebenfalls zu dem ersten Zeitpunkt innerhalb des ersten Zeitraums t1 können die piezoelektrischen Elemente 20, 21, 22 der dritten Anordnung eingerichtet sein, Ultraschall für eine Ultraschallmessung zu erzeugen.

Die piezoelektrischen Elemente 10, 11, 12, 13 der zweiten Anordnung können in einer ersten Ebene angeordnet sein. Die piezoelektrischen Elemente 20, 21, 22 der dritten Anordnung können in einer zweiten Ebene angeordnet sein. Die erste Ebene und die zweite Ebene können voneinander beabstandet sein. Die erste Ebene und die zweite Ebene können (jeweils) parallel zu einer von der Wundauflage 100 definierten Ebene orientiert sein.

Die piezoelektrischen Elemente 10, 11, 12, 13 der zweiten Anordnung können versetzt zu den piezoelektrischen Elemente 20, 21, 22 der dritten Anordnung angeordnet sein. Zwischen zwei benachbarten piezoelektrischen Elementen der piezoelektrischen Elemente 10, 11, 12, 13 der zweiten Anordnung kann in der ersten Ebene ein Abstand vorliegen, der zumindest die Größe einer Abmessung eines piezoelektrischen Elements der piezoelektrischen Elemente 20, 21, 22 der dritten Anordnung besitzt. Die Abmessung kann eine Breite und/oder Länge sein. Insbesondere ist die Breite und/oder Länge in einer parallel zu einer von der Wundauflage 100 definierten Ebene orientiert.

Die Ultraschallmessung innerhalb des ersten Zeitraums kann als erste Ultraschallmessung bezeichnet werden.

In Fig. 6b ist die Wundauflage 100 innerhalb eines zweiten Zeitraums t2 dargestellt. Die Ultraschallmessung innerhalb des zweiten Zeitraums t2 kann als zweite oder weitere Ultraschallmessung bezeichnet werden. Die Position oder die Lage der Wundauflage 100 kann von Beginn des ersten Zeitraums t1 bis Ende des zweiten Zeitraums t2 unverändert auf dem Hautabschnitt 210 sein. In dem zweiten Zeitraum t2, in dem die zweite Ultraschallmessung durchführbar ist, hat sich die Wunde 300 in dem Hautabschnitt 210 analog oder gleich zu der Veränderung der Wunde 300 wie mit Blick auf die Figs. 4a bis 4d beschrieben verändert.

Durch die erste und zweite Ultraschallmessung lässt sich eine Veränderung der Wunde 300 ermitteln. Beispielsweise ist ein Heilungsfortschritt und/oder eine Heilungskomplikation ermittelbar.

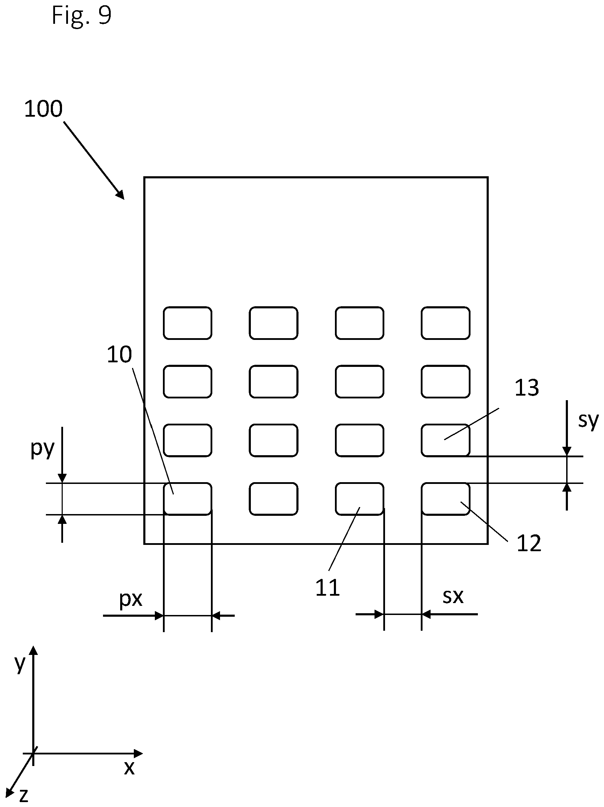

Fig. 9 zeigt schematisch eine Wundauflage 100 in einer Draufsicht. Für eine bessere Erkennbarkeit sind in Fig. 9 lediglich piezoelektrische Elemente 10, 11, 12, 13 in der Wundauflage 100 gezeigt. Die Wundauflage kann jede hierin offenbarte Wundauflage 100 sein. Die piezoelektrischen Elemente können jede hierin offenbarten piezoelektrischen Elemente sein.

Zumindest eine Abmessung eines piezoelektrischen Elements 10 kann zwischen 0,1 mm und 10,0 mm. Die Abmessung kann eine Breite px und/oder eine Länge py sein. Die Länge und/oder die Breite kann sich in einer von der Wundauflage 100 definierten Ebene erstrecken.

Bevorzugt ist die Breite px und/oder Länge py zumindest einiger der piezoelektrischen Elemente 10, 11, 12, 13 der Wundauflage 100 gleich. Besonders bevorzugt ist die Breite px und/oder Länge py aller piezoelektrischer Elemente 10, 11, 12, 13 der Wundauflage 100 gleich.

Die Breite px und/oder Länge py von zumindest zwei der piezoelektrischen Elemente 10, 11, 12, 13 kann unterschiedlich sein.

Die Breite von zumindest einem, bevorzugt von allen, der piezoelektrischen Elemente 10, 11, 12, 13 kann größer als die Länge von zumindest einem, bevorzugt allen, der piezoelektrischen Elemente 10, 11, 12, 13. Die Länge von zumindest einem, bevorzugt von allen, der piezoelektrischen Elemente 10, 11, 12, 13 kann größer als die Breite von zumindest einem, bevorzugt allen, der piezoelektrischen Elemente 10, 11, 12, 13.

Ein Abstand sx, sy zwischen zwei benachbarten piezoelektrischen Elementen 10, 11, 12, 13 kann zwischen 0,25 mm und 20,0 mm liegen. Der Abstand sx, sy kann ein Abstand sx in eine erste Richtung (x-Richtung) in einer von der Wundauflage 100 definierten Ebene sein und/oder der Abstand sx, sy kann ein Abstand sy in eine zweite Richtung (y-Richtung) in einer von der Wundauflage 100 definierten Ebene sein. Die erste Richtung und die zweite Richtung können abgewinkelt zueinander sein. Insbesondere ist die erste Richtung senkrecht zu der zweiten Richtung.

Der Abstand sx in der ersten Richtung zwischen jeweils zwei benachbarten piezoelektrischen Elementen 10, 11, 12, 13 aller piezoelektrischer Elemente 10, 11, 12, 13 kann gleich oder unterschiedlich sein. Der Abstand sy in der zweiten Richtung zwischen jeweils zwei benachbarten piezoelektrischen Elementen 10, 11, 12, 13 aller piezoelektrischer Elemente 10, 11, 12, 13 kann gleich oder unterschiedlich sein.

Der Abstand sx in der ersten Richtung zwischen zwei benachbarten piezoelektrischen Elementen 10, 11, 12, 13 kann größer oder kleiner sein als der Abstand sy in der zweiten Richtung zwischen zwei benachbarten piezoelektrischen Elementen 10, 11, 12, 13.

### BEZUGSZEICHENLISTE

- 100: Wundauflage
- 10: piezoelektrisches Element
- 11: piezoelektrisches Element
- 12: piezoelektrisches Element
- 13: piezoelektrisches Element
- 15: Verbindungsstruktur
- 20: piezoelektrisches Element
- 21: piezoelektrisches Element
- 22: piezoelektrisches Element
- 30: piezoelektrisches Element
- 31: piezoelektrisches Element
- 32: piezoelektrisches Element
- 40: Steuereinheit
- 50: Datenübertragungseinrichtung
- 60: Energieversorgungseinrichtung
- 80: Kontaktschicht
- 81: Schicht
- 82: Zwischenschicht
- 83: Deckschicht
- 85: zentraler Abschnitt
- 90: Klebeschicht

- 200: Körper
- 210: Hautabschnitt
- 300: Wunde
- 310: Biofilm
- 500: Auswerteeinheit

- t1: Zeitraum
- t2: Zeitraum
- t3: Zeitraum

- px: Abmessung
- py: Abmessung
- sx: Abstand
- sy: Abstand

## Patentansprüche

1. Wundauflage (100) zur Behandlung einer Wunde (300), wobei:
- die Wundauflage (100) auf einen Hautabschnitt (210) eines Menschen oder eines Tieres aufbringbar ist, wobei der Hautabschnitt (210) die Wunde (300) umfasst;
- die Wundauflage (100) eine erste Anordnung von piezoelektrischen Elementen (30, 31, 32) umfasst, wobei zumindest einige der piezoelektrischen Elemente (30) eingerichtet sind, Ultraschall für eine Ultraschallbehandlung der Wunde (300) zu erzeugen.

2. Wundauflage nach Anspruch 1, wobei die piezoelektrischen Elemente (30) so steuerbar sind, dass höchstens einige der piezoelektrischen Elemente (30) Ultraschall für die Ultraschallbehandlung der Wunde (300) erzeugen; und/oder wobei die piezoelektrischen Elemente (30) so steuerbar sind, dass nur definierte der piezoelektrischen Elemente (30) Ultraschall für die Ultraschallbehandlung der Wunde (300) erzeugen.

3. Wundauflage nach Anspruch 1 oder 2, wobei die piezoelektrischen Elemente (30) so steuerbar sind, dass zumindest zwei der piezoelektrischen Elemente (30) Ultraschall mit unterschiedlicher Leistung für die Ultraschallbehandlung der Wunde (300) erzeugen;

4. Wundauflage nach einem der vorhergehenden Ansprüche, wobei die Wundauflage eine Wundauflage zur Überwachung und Behandlung einer Wunde (300) ist, wobei die Wundauflage (100) eine zweite Anordnung von piezoelektrischen Elementen (10, 11, 12, 13) umfasst, wobei zumindest einige der piezoelektrischen Elemente (10) der zweiten Anordnung eingerichtet sind, Ultraschall für eine Ultraschallmessung des Hautabschnitts (210) zu erzeugen, wobei auf Grundlage der Ultraschallmessung des Hautabschnitts (210) eine oder mehrere Eigenschaften der Wunde (300) ermittelbar ist.

5. Wundauflage nach Anspruch 4, wobei zumindest einige der piezoelektrischen Elemente (10) der zweiten Anordnung eingerichtet sind, Ultraschall für eine erste Ultraschallmessung des Hautabschnitts (210) innerhalb eines ersten Zeitraums (t1) zu erzeugen und Ultraschall für eine zweite Ultraschallmessung des Hautabschnitts (210) innerhalb eines zweiten Zeitraums (t2) zu erzeugen, wobei auf Grundlage der ersten Ultraschallmessung und der zweiten Ultraschallmessung die eine oder mehreren Eigenschaften der Wunde (300) ermittelbar ist.

6. Wundauflage nach Anspruch 4 oder 5, wobei die eine oder mehreren Eigenschaften der Wunde (300) eine Fläche der Wunde, eine Tiefe der Wunde, ein Heilungsfortschritt der Wunde, eine Heilungskomplikation der Wunde, eine Zustandsänderung der Wunde, eine Dichte, ein Wassergehalt, eine Zusammensetzung, Gewebeschichten und/oder ein mikrobieller Zustand der Wunde ist.

7. Wundauflage nach einem der Ansprüche 4 bis 6, wobei:
- die piezoelektrischen Elemente (10) der zweiten Anordnung eingerichtet sind, Ultraschall für die Ultraschallmessung zu erzeugen und zu empfangen, insbesondere wobei die piezoelektrischen Elemente (10) der zweiten Anordnung eingerichtet sind, innerhalb eines Zeitraums (t1, t2) zeitlich versetzt Ultraschall zu erzeugen und Ultraschall zu empfangen; und/oder
- die Wundauflage (100) eine dritte Anordnung von piezoelektrischen Elementen (20, 21, 22) umfasst, wobei die piezoelektrischen Elemente (10) der zweiten Anordnung eingerichtet sind, Ultraschall für die Ultraschallmessung zu erzeugen, und die piezoelektrischen Elemente (20) der dritten Anordnung eingerichtet sind, Ultraschall für die Ultraschallmessung zu empfangen, insbesondere wobei innerhalb eines ersten Zeitraums die piezoelektrischen Elemente (10) der zweiten Anordnung eingerichtet sind, Ultraschall zu erzeugen, und die piezoelektrischen Elemente (20) der dritten Anordnung eingerichtet sind, Ultraschall zu empfangen, und innerhalb eines zweiten Zeitraums die piezoelektrischen Elemente (10) der zweiten Anordnung eingerichtet sind, Ultraschall zu empfangen, und die piezoelektrischen Elemente (20) der dritten Anordnung eingerichtet sind, Ultraschall zu senden; und/oder
- die Wundauflage (100) eine dritte Anordnung von piezoelektrischen Elementen (20, 21, 22) umfasst, wobei die piezoelektrische Elemente (10) der zweiten Anordnung in einer ersten Ebene angeordnet sind und die piezoelektrischen Elemente (20) der dritten Anordnung in einer zweiten Ebene angeordnet sind, wobei die erste Ebene und die zweite Ebene übereinander liegend orientiert sind, bevorzugt wobei die piezoelektrischen Elemente (10) der zweiten Anordnung eingerichtet sind, Ultraschall für die Ultraschallmessung zu erzeugen, und, insbesondere gleichzeitig, die piezoelektrischen Elemente (20) der dritten Anordnung eingerichtet sind, Ultraschall für die Ultraschallmessung zu empfangen; und/oder
- die piezoelektrischen Elemente (30) der ersten Anordnung in einer ersten Ebene angeordnet sind und die piezoelektrischen Elemente (10, 20) der zweiten und/oder dritten Anordnung in einer zweiten Ebene angeordnet sind, wobei die erste Ebene und die zweite Ebene übereinander liegend orientiert sind; und/oder
- die piezoelektrischen Elemente (30) der ersten Anordnung in einer Ebene mit den piezoelektrischen Elementen (10, 20) der zweiten und/oder dritten Anordnung angeordnet sind.

8. Wundauflage nach einem der Ansprüche 4 bis 7, wobei die piezoelektrischen Elemente (30) der ersten Anordnung eingerichtet sind, Ultraschall mit einer gleichen oder höheren Leistung zu erzeugen als die piezoelektrischen Elemente (10, 20) der zweiten und/oder dritten Anordnung.

9. Wundauflage nach einem der Ansprüche 4 bis 8, wobei die piezoelektrischen Elemente (30) der ersten Anordnung eingerichtet sind, Ultraschall für die Ultraschallbehandlung der Wunde (300) auf Grundlage der einen oder mehreren Eigenschaften der Wunde (300) zu erzeugen.

10. Wundauflage nach einem der vorhergehenden Ansprüche, wobei die Wundauflage (100):
- eine Steuereinheit (40) umfasst, wobei die Steuereinheit (40) eingerichtet ist, die piezoelektrischen Elemente (10, 20, 30) zu steuern, insbesondere die Erzeugung von Ultraschall für die Ultraschallbehandlung zu steuern und/oder die Erzeugung und/oder das Empfangen von Ultraschall für die Ultraschallmessung durch die piezoelektrischen Elemente (10, 20); und/oder
- eine Datenübertragungseinrichtung (50) umfasst, wobei die Datenübertragungseinrichtung (50) eingerichtet ist, Daten der Ultraschallmessung an eine Auswerteeinrichtung (500) zu übertragen.

11. Wundauflage nach einem der vorhergehenden Ansprüche, wobei die Wundauflage (100) eine Energieversorgungseinrichtung (60) umfasst, wobei die Energieversorgungseinrichtung (60) eingerichtet ist, Energie für die Wundauflage (100) bereitzustellen, insbesondere wobei die Energieversorgungseinrichtung (60) einen Energiespeicher umfasst, durch den gespeicherte Energie für die Wundauflage (100) bereitstellbar ist; und/oder wobei die Energieversorgungseinrichtung (60) eingerichtet ist, Energie kontaktbehaftet oder kontaktlos zu empfangen, insbesondere durch elektromagnetische Strahlung zu empfangen.

12. Wundauflage nach einem der vorhergehenden Ansprüche, wobei:
- die erste, zweite und/oder dritte Anordnung von piezoelektrischen Elementen (10, 20, 30) teil- oder vollelastisch, teil- oder vollbiegsam, und/oder teil- oder vollflexibel ist; und/oder
- die Wundauflage (100) so ausgebildet ist, dass bei einem Aufbringen der Wundauflage (100) auf den Hautabschnitt (210) zumindest einige der piezoelektrischen Elemente (10, 20, 30) den Hautabschnitt (210) kontaktieren, insbesondere direkt kontaktieren; oder wobei die Wundauflage (100) eine Kontaktschicht (80), insbesondere eine Hydrogelschicht umfasst, die den Hautabschnitt (210) kontaktiert, wenn die Wundauflage (100) auf den Hautabschnitt (100) aufgebracht ist; und/oder
- die Wundauflage (100) eine Schicht (81) umfasst, wobei die Schicht (81) zumindest einige der piezoelektrischen Elemente (10, 20, 30) bedeckt oder aufnimmt, bevorzugt wobei die Schicht (81) ein Elastomer, insbesondere ein Silikon, umfasst; und/oder
- die Wundauflage (100) eine Zwischenschicht (82) umfasst, wobei die Zwischenschicht (82) ein absorbierendes Material und/oder ein bioaktives Material umfasst; und/oder
- die Wundauflage (100) eine Deckschicht (83) umfasst, wobei die Deckschicht (83) die Wundauflage (100) zumindest teilweise, insbesondere vollständig, bedeckt; und/oder
- die Wundauflage (100) eine zumindest teilweise umlaufende, insbesondere vollständig umlaufende, Klebeschicht (90) umfasst.

13. Wundauflage nach einem der vorhergehenden Ansprüche, wobei:
- die piezoelektrischen Elemente (10, 20, 30) eine Abmessung (px, py), insbesondere eine Breite und/oder eine Länge, zwischen 0,1 mm und 10,0 mm, bevorzugt zwischen 0,2 mm und 8,0 mm, bevorzugter zwischen 0,4 mm und 6,0 mm, bevorzugter zwischen 0,6 mm und 4,0 mm, bevorzugter zwischen 0,8 mm und 2,0 mm, bevorzugter zwischen 0,9 mm und 1,5 mm, aufweisen; und/oder
- die piezoelektrischen Elemente (10, 20) eine Fläche zwischen 0,01 mm² und 100,0 mm², bevorzugt zwischen 0,05 mm² und 80,0 mm², bevorzugter zwischen 0,10 mm² und 60,0 mm², bevorzugter zwischen 0,30 mm² und 40,0 mm², bevorzugter zwischen 0,50 mm² und 20,0 mm², bevorzugter zwischen 0,60 mm² und 10,0 mm², bevorzugter zwischen 0,80 mm² und 5,0 mm², aufweisen; und/oder
- zwischen zwei benachbarten piezoelektrischen Elementen (10, 20) ein Abstand (sx, sy) zwischen 0,25 mm und 20,0 mm, bevorzugt zwischen 0,25 mm und 15,0 mm, bevorzugter zwischen 0,25 mm und 10,0 mm, bevorzugter zwischen 0,25 mm und 5,0 mm, bevorzugter zwischen 0,5 mm und 5,0 mm, bevorzugter zwischen 0,5 mm und 1,0 mm, vorliegt.

14. Wundauflage nach einem der vorhergehenden Ansprüche, wobei zumindest eines der piezoelektrischen Elemente (10, 20, 30) die Wunde (300) überlagert und zumindest eines der piezoelektrischen Elemente (10, 20, 30) die Wunde (300) nicht überlagert, wenn die Wundauflage (100) auf dem Hautabschnitt (210) aufgebracht ist.

15. System mit einer Wundauflage (100) nach einem der Ansprüche 4 bis 14 und einer Auswerteeinheit (500), wobei die Auswerteeinheit (500) eingerichtet ist, auf Grundlage der Ultraschallmessung des Hautabschnitts (210) die eine oder mehreren Eigenschaften der Wunde (300) zu ermitteln.
